# EUROPEAN PATENT APPLICATION

(11) **EP 4 316 529 A1**
(43) Date of publication of application: **07.02.2024**
(21) Application number: 22775645.9
(22) Date of filing: 23.03.2022
(51) Int. Cl.: A61K 49/00, C08B 37/16

(54) **CONTRAST IMAGING COMPOSITION FOR CANCER**

(30) Priority: 24.03.2021 JP 2021049967
(71) Applicant: Astellas Pharma Inc., Chuo-ku Tokyo 103-8411 (JP); Mie University, Tsu-shi, Mie 514-8507 (JP)
(72) Inventor: TERANISHI, Katsunori, Tsu-shi, Mie 514-8507 (JP); FUSHIKI, Hiroshi, Tokyo 103-8411 (JP)
(74) Representative: Hoffmann Eitle
(86) International application number: PCT/JP2022/013353
(87) International publication number: WO 2022/202863

(57) **Abstract**

An object of the present invention is to provide a contrast agent capable of specifically identifying a cancer tissue. When a composition for contrast imaging of cancer containing a compound of the present invention is administered, a cancer tissue can be imaged with near-infrared light.

## Description

### TECHNICAL FIELD

The present invention relates to a contrast agent for cancer. Particularly, the present invention relates to a composition for contrast imaging of cancer containing a cyclodextrin-bonded indocyanine compound.

### BACKGROUND ART

Cancer is the leading cause of death in Japan, and also globally, is a disease whose mortality is increasing, and that is always highly ranked as the cause of death. A surgical treatment for cancer has been widespread as a therapeutic method capable of complete cure since a long time ago, but tumor location is not able to be accurately identified, and recurrence/aggravation due to insufficient excision is a serious medical problem. Besides, partial resection is selected in more cases owing to improvement of surgical precision in recent years, but there is always a risk of insufficient excision, and accurate identification of tumor location has been more and more expected.

As a pre-operative location identification method for cancer surgery, non-invasive methods such as computerized tomography (CT), magnetic resonance imaging (MRI), and positron emission tomography (PET) imaging are employed, and these methods play a significant role in pre-operative diagnosis/consideration of surgical method. These methods do not, however, provide information on actual tumor location during surgery, and the tumor location is confirmed only visually by a surgeon during surgery, and hence, these methods do not lead to improvement of incomplete resection due to visual difficulties caused by bleeding and near tissues.

Intraoperative guided diagnosis using fluorescence images is a technique having been started to be employed for supporting visual identification of tumor location. For example, in cystoscopic diagnosis of bladder cancer, gross diagnosis under white light, and observation of red fluorescence under blue light irradiation of protoporphyrin IX generated by administration of 5-aminolaevulinic acid (ALA) or hexyl-5-aminolaevulinic acid ester (H-ALA) (photodynamic diagnosis) are carried out. A site of a bladder cancer tissue is determined through these diagnoses, and a cancer tissue can be more easily determined by the photodynamic diagnosis through administration of ALA or H-ALA than by the gross diagnosis under white light, and hence, a risk of insufficient resection of a cancer tissue in excision of the cancer tissue can be reduced so as to reduce recurrence of the cancer. Currently, H-ALA is commercially available from Photocure (Norway, https://www.photocure.com/) under the trade name Hexvix (Europe) or Cysview (U.S. and Canada) (NPL 1). Besides, for the purposes of improving the diagnostic ability of the photodynamic diagnosis through administration of ALA or H-ALA, for example, methods of dendrimerization of H-ALA (NPL 2), nanoparticulation (NPL 3), and the like have been reported.

Besides, a normal site and an affected site in a kidney tissue are distinguished from each other by a technique of CT image analysis, or a pathological examination through excision/dyeing/microscopic observation. Intraoperative identification of an affected site of kidney tissue during surgery is also carried out visually by a human.

Besides, owing to technical innovation in recent years, fluorescence imaging technique using a near-infrared region wavelength that can easily transmit a biological component has been spread, and its medical application has started to be spread in various regions. The near-infrared region wavelength cannot be detected by human eyes but can be detected with a CCD camera or the like, and hence is one of effective means as a role in supporting visibility during surgery.

As a fluorescent reagent utilizing the near-infrared region wavelength, indocyanine green (ICG) is known, and is put on the market as an agent for testing hepatic/circulatory function, a fluorescent angiographic agent, and an agent for identifying a sentinel lymph node. It is noted that a sentinel lymph node is a lymph node that a cancer cell having entered a lymphatic vessel from the primary tumor first reaches, and is a lymph node having the highest possibility of metastasis. A method in which such a lymph node is identified/excised to determine through pathological diagnosis whether or not cancer has been metastasized is designated as sentinel lymph node biopsy (SNB). The ICG is used for identifying a sentinel lymph node, but does not have an imaging effect specific to tumor due to its high protein binding ability, and it is not easy to image tumor itself by employing the near-infrared fluorescence technology (NPL 4).

Currently, drug development with expectation of tumor fluorescence imaging is carried out in the world, but there exists no drug usable in a medical environment yet.

PTL1 describes a specific cyclodextrin-bonded indocyanine compound as a fluorescent reagent utilizing the near-infrared region wavelength, and describes that a diagnostic composition containing the compound is applicable to fundus angiography, assessment of cerebral circulation, intraoperative imaging in brain surgery, identification of a sentinel lymph node in cancer (such as breast cancer, esophageal cancer, stomach cancer, colorectal cancer, prostate cancer, or skin cancer), assessment of lymphedema, intraoperative cholangiography, tumor marking, coronary angiography, abdominal angiography (of hepatic artery, abdominal aorta, gastrointestinal blood flow, and the like), and the like, for which imaging has been conventionally performed.

### CITATION LIST

### PATENT LITERATURE

PTL 1: U.S. Patent Publication No. 2012/0302881

### NON PATENT LITERATURE

NPL 1: Michael Rink et al., European Urology, 2013, 64, 624-638
NPL 2: Francois, Aurelie et al., BJU International, 2012, 110(11c), E1155-1162
NPL 3: Heuck Gesine et al., Journal of Porphyrins and Phthalocyanines, 2012, 16(7-8), 878-884
NPL 4: Shenglin Luo, Erlong Zhang, Yongping Su, Tianmin Cheng, Chunmeng Shi. A review of NIR dyes in cancer targeting and imaging, Biomaterials, 2011, 32 (29), 7127-7138

### SUMMARY OF INVENTION

### TECHNICAL PROBLEM

Since the photodynamic diagnosis using ALA or H-ALA is carried out by fluorescence detection of red light through irradiation with blue light, the entire bladder looks blue or blue purple, and on this background, a cancer tissue is visually recognized as red, and therefore, diagnosis of deep tissue and determination of a boundary between a normal tissue and the cancer tissue cannot be clearly made (A. Kolodziej, W. Krajewski, M. Matuszewski, K. Tupikowski. Review of current optical diagnostic techniques for non-muscle-invasive bladder cancer, Cent European J Urol. 2016; 69: 150-156). In addition, blue light is poor in biological tissue transmittance, and it is apprehended that tumor embedded in surrounding tissues may not be detected.

Besides, identification (imaging) of a sentinel lymph node using ICG or the like is useful for providing structural information on a lymphatic vessel-lymph node connected to tumor, but is not means for observing the tumor itself.

In general, it is difficult to image or discriminate tumor itself with a near-infrared fluorescent reagent even with the technical innovation of recent years, and although drug development with expectation of tumor fluorescence imaging is being carried out in the world, there exists no drug usable in a medical environment yet.

In consideration of these circumstances, an object of the present invention is to develop a contrast agent capable of specifically identifying or discriminating a cancer tissue.

### SOLUTION TO PROBLEM

The present inventors have made earnest studies to solve the above-described problem, resulting in finding that a cancer tissue can be specifically identified or discriminated through near-infrared irradiation by using a compound in which cyclodextrin is bonded to an indocyanine compound. Specifically, the present inventors actually administered the compound in which cyclodextrin is bonded to an indocyanine compound, and performed near-infrared irradiation, and it was thus confirmed that a normal tissue did not emit near-infrared fluorescence derived from this compound but a cancer tissue emitted near-infrared fluorescence, and that a cancer tissue of a certain type of cancer emits the near-infrared fluorescence derived from this compound only weakly as compared with a normal tissue but the normal tissue emitted the near-infrared fluorescence, and thus, the present invention was accomplished.

Specifically, the present inventors found the following: When a cyclodextrin-bonded indocyanine compound that emits near-infrared fluorescence through near-infrared irradiation for discriminating cancer, for example, a bladder cancer tissue from a normal tissue is intravenously administered (systemically administered) or intravesically administered to a bladder cancer mouse model, and the resultant model is subjected to near-infrared irradiation, a normal tissue in the bladder does not emit near-infrared fluorescence derived from this compound but a cancer tissue emits the near-infrared fluorescence. Besides, the present inventors also found the following: When a cyclodextrin-bonded indocyanine compound is intravenously administered (systemically administered) to mouse models having stomach cancer, peritoneal cancer originating from renal cell cancer or peritoneal dissemination originating from stomach cancer, and esophageal cancer, and the resultant models are subjected to near-infrared irradiation, these cancer tissues emit stronger near-infrared fluorescence than surrounding normal tissues. The present inventors also found the following: When a cyclodextrin-bonded indocyanine compound is intravenously administered (systemically administered) to mouse models having kidney cancer, lung cancer originating from renal cell cancer, and liver cancer originating from renal cell cancer, and the resultant models are subjected to near-infrared irradiation, emission of near-infrared fluorescence from these cancer tissues are weaker than emission of near-infrared fluorescence from surrounding normal tissues, and thus, the cancer tissues can be distinguished from the normal tissues. Besides, the present inventors administered a cyclodextrin-bonded indocyanine compound to a living body, and obtained a near-infrared fluorescence image of the kidney, and thus, extracted an abnormal area of renal cells on a micro level, and extracted an abnormal area of kidney tissues on a macro level. Besides, the present inventors found the following: When a cyclodextrin-bonded indocyanine compound is topically administered to a desired cancer tissue, such as colorectal cancer, breast cancer, skin cancer, head and neck cancer, or brain cancer, and the resultant is subjected to near-infrared irradiation, the compound is retained in the cancer tissue, and the cancer tissue emits near-infrared fluorescence.

The present invention encompasses, but is not restricted to, at least the following aspects:
[1] A composition for contrast imaging of cancer, containing a cyclodextrin-bonded indocyanine compound represented by the following formula, or a pharmaceutically acceptable salt thereof: 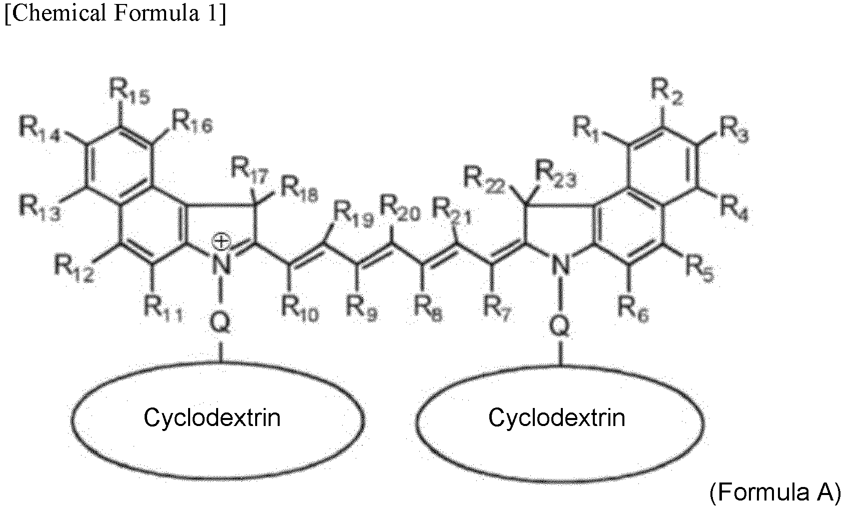
   wherein two Qs are each capable of being independently selected, are optionally the same or different, and are each * 1-(CH₂)a-CO-NH-(CH₂)b-*3, wherein a is an integer of 2 or more and 6 or less, b is an integer of 2 or more and 6 or less, * 1 in each Q is N of the indocyanine skeleton, and *3 is a bonding portion formed by replacing any one of three OH groups of any D-glucose contained in cyclodextrin with -O-;
   R1 to R23 each independently are a hydrogen atom, an alkyl group, an aryl group, a halogen atom, an alkoxyl group, an amino group, a carboxyl group, a formyl group, a sulfonyl group, a sulfonic acid group, a phosphoric acid group, an alkyloxycarbonyl group, an aryloxycarbonyl group, an alkylcarbonyl group, an arylcarbonyl group, or a heterocycle, R1 to R23 each independently are unsubstituted or substituted with a substituent, when the substituent is carboxylic acid, sulfonic acid, or phosphoric acid, a hydrogen ion is optionally dissociated from the substituent to be replaced with a metal ion;
   R8 and R9 optionally together form a cyclic structure of CH₂, CH₂CH₂, CH₂CH₂CH₂, or CH₂CH₂CH₂CH₂, and one or more hydrogen atoms of the cyclic structure is optionally replaced with an aryl group, a halogen atom, an alkoxy group, an amino group, a carboxyl group, a formyl group, a sulfonyl group, a sulfonic acid group, a phosphoric acid group, an alkyloxycarbonyl group, an aryloxycarbonyl group, an alkylcarbonyl group, an arylcarbonyl group, or a heterocycle.
[2] The composition according to [1],
   wherein the cyclodextrin-bonded indocyanine compound is a compound represented by the following formula, or a pharmaceutically acceptable salt thereof: 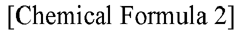 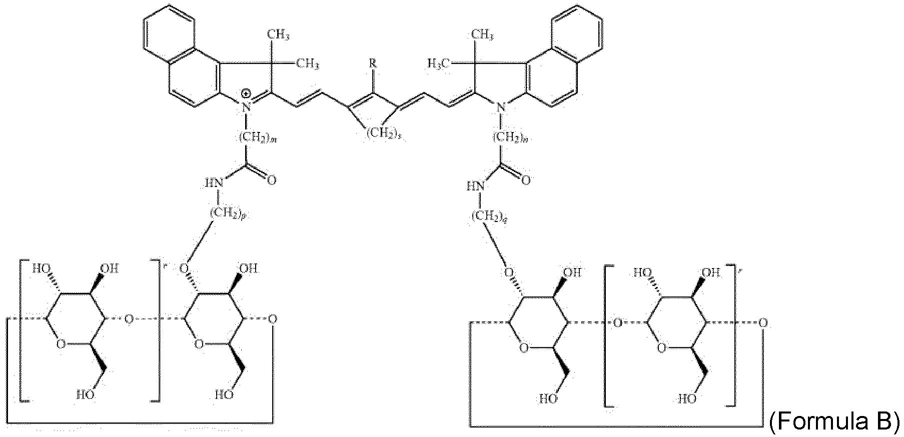
   wherein m, n, p, and q are each independently an integer of 2 or more and 6 or less, r is an integer of 5 or more and 7 or less, s is an integer of 0 or more and 4 or less, and R is a hydrogen atom, an alkyl group, an aryl group, a halogen atom, an alkoxy group, an amino group, a carboxyl group, a formyl group, a sulfonyl group, a sulfonic acid group, an alkyloxycarbonyl group, an aryloxycarbonyl group, an alkylcarbonyl group, an arylcarbonyl group, or a heterocycle.
[3] The composition according to [1] or [2], wherein the cyclodextrin-bonded indocyanine compound is a compound represented by the following formula, or a pharmaceutically acceptable salt thereof: 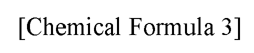 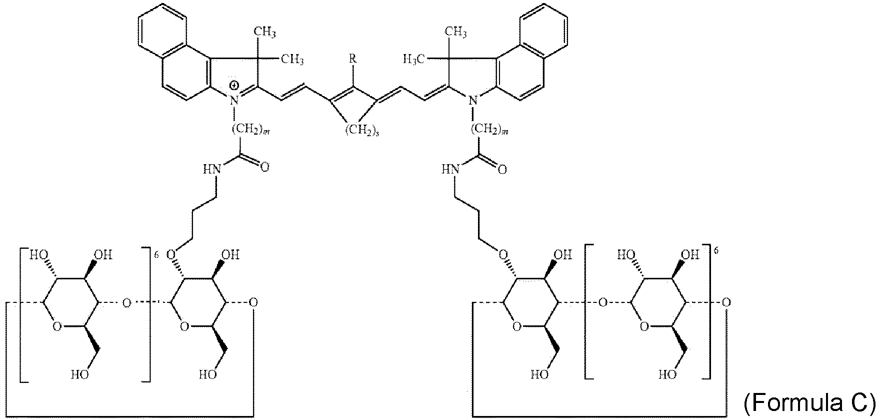 wherein m and n are each independently an integer of 2 or more and 6 or less, s is an integer of 0 or more and 4 or less, and R is a hydrogen atom, an alkyl group, an aryl group, a halogen atom, an alkoxy group, an amino group, a carboxyl group, a formyl group, a sulfonyl group, a sulfonic acid group, an alkyloxycarbonyl group, an aryloxycarbonyl group, an alkylcarbonyl group, an arylcarbonyl group, or a heterocycle.
[4] The composition according to any one of [1] to [3], wherein the cyclodextrin-bonded indocyanine compound contains a compound represented by the following formula, or a pharmaceutically acceptable salt thereof: 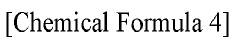 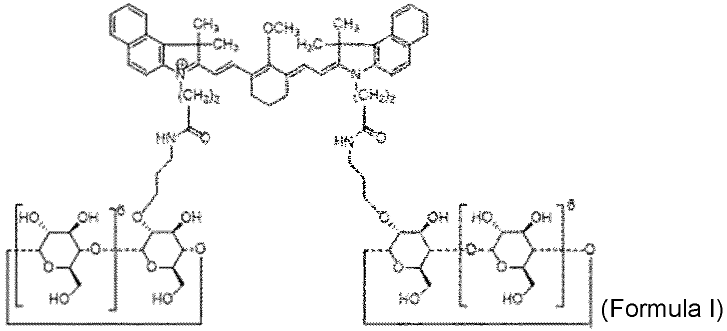 or 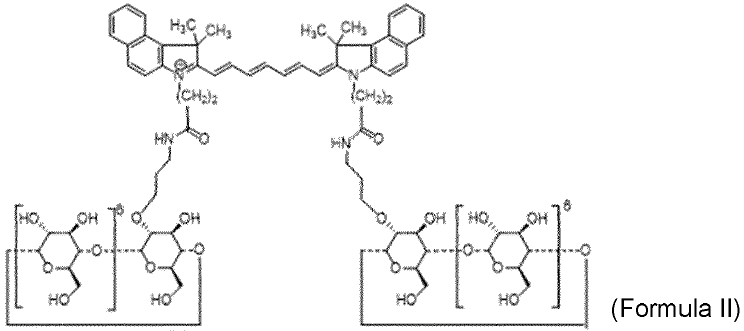
[5] The composition according to any one of [1] to [4], in the form of a liquid.
[6] The composition according to any one of [1] to [5], for use in imaging bladder cancer, kidney cancer, lung cancer, liver cancer, stomach cancer, peritoneal cancer, peritoneal dissemination, or esophageal cancer.
[7] The composition according to any one of [1] to [5], for use in imaging breast cancer, colorectal cancer, skin cancer, head and neck cancer, or brain cancer.
[8] The composition according to any one of [1] to [6], for systemic administration.
[9] The composition according to any one of [1] to [5], and [7], for topical administration.
[10] The composition according to any one of [1] to [5], for use in imaging bladder cancer, and for intravesical administration via the urethra.
[11] The composition according to any one of [1] to [5], for use in imaging bladder cancer, kidney cancer, lung cancer, liver cancer, stomach cancer, peritoneal cancer, peritoneal dissemination, or esophageal cancer, and for systemic administration.
[12] The composition according to any one of [1] to [5], for use in imaging breast cancer, colorectal cancer, skin cancer, head and neck cancer, or brain cancer, and for topical administration.
[13] A cancer imaging method, including administering the composition according to any one of [1] to [5] to a subject.
[14] The cyclodextrin-bonded indocyanine compound or a pharmaceutically acceptable salt thereof according to any one of [1] to [4], for imaging cancer.
[15] Use of the cyclodextrin-bonded indocyanine compound or a pharmaceutically acceptable salt thereof according to any one of [1] to [4] for imaging cancer.
[16] Use of the cyclodextrin-bonded indocyanine compound or a pharmaceutically acceptable salt thereof according to any one of [1] to [4] for producing a composition for contrast imaging of cancer.

It is noted that a "subject" is a human or another animal requiring contrast imaging of cancer, and is a human requiring contrast imaging of cancer in one aspect.

In the cyclodextrin-bonded indocyanine compound of the present invention, at least a part of a naphthyl moiety of indocyanine can be included by cyclodextrin in a molecule, such a compound is in an isomerization equilibrium state in an aqueous solution, and can be in an equilibrium state between inclusion type and non-inclusion type.

### ADVANTAGEOUS EFFECTS OF INVENTION

According to the present invention, a cancer tissue can be specifically identified or discriminated by near-infrared irradiation. Specifically, according to the present invention, tumor tissues including bladder cancer, kidney cancer, stomach cancer, liver cancer, lung cancer, peritoneal dissemination, peritoneal cancer, esophageal cancer, colorectal cancer, breast cancer, skin cancer, head and neck cancer, brain cancer or the like can be discriminated based on observation of near-infrared fluorescence emitted by near-infrared irradiation.

When a compound of the present invention is administered to a subject, the compound is retained in a cancer tissue, and therefore, when the compound of the present invention is systemically administered to a subject having cancer such as bladder cancer, stomach cancer, peritoneal dissemination, peritoneal cancer, or esophageal cancer, or topically administered to a cancer tissue such as colorectal cancer, breast cancer, skin cancer, head and neck cancer, or brain cancer, the cancer tissue emits near-infrared fluorescence by near-infrared irradiation.

In other words, when a composition for contrast imaging of cancer of the present invention is administered into a tumor of an individual having cancer, it can be used as a fluorescence imaging method capable of accurate identification of tumor location during cancer surgery because retention selectivity in tumor is higher than that of a known near-infrared fluorescence contrast agent, indocyanine green, and a background value in a normal tissue is set low.

Besides, when the compound of the present invention is administered to a subject having a certain type of cancer, such as kidney cancer, liver cancer, or lung cancer, a cancer tissue and a normal tissue can be distinguished from each other by near-infrared irradiation. Specifically, when the compound of the present invention is systemically administered to a subject, near-infrared fluorescence emitted from the cancer tissue of the certain type of cancer is weaker than near-infrared light emitted from a surrounding normal tissue, and hence, the cancer tissue can be distinguished from the normal tissue, and thus, the tumor location can be accurately identified during cancer surgery or the like.

In one aspect, the present invention can provide a discrimination guide technique to be employed in a surgical resection operation of a cancer tissue, or the like.

### BRIEF DESCRIPTION OF DRAWINGS

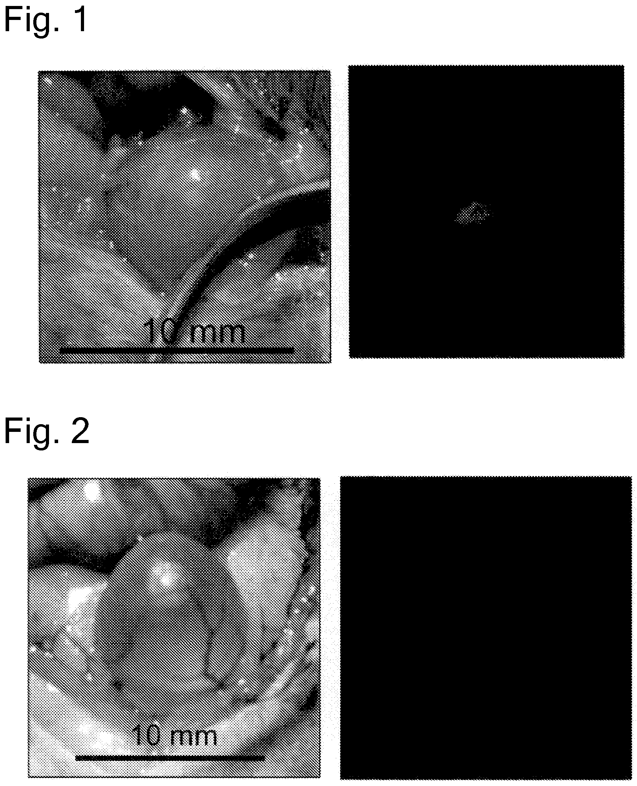

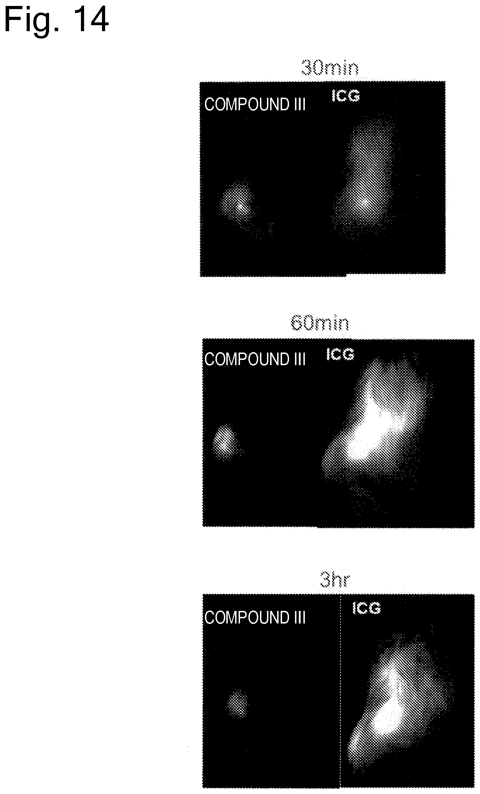

[Fig. 1] Fig. 1 illustrates photographs of the bladder taken one day after tail vein administration of a composition for contrast imaging of cancer containing a compound III to an MB49 mouse bladder cancer model (left: under white light, right: near-infrared fluorescence image).
[Fig. 2] Fig. 2 illustrates photographs of the bladder taken one day after tail vein administration of the composition for contrast imaging of cancer containing the compound III to a normal mouse (left: under white light, right: near-infrared fluorescence image).

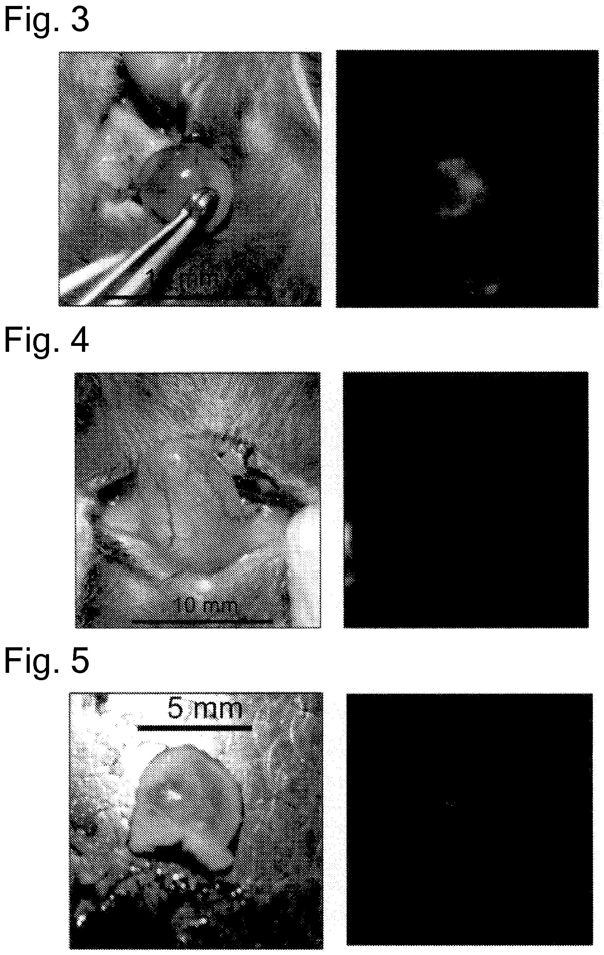

[Fig. 3] Fig. 3 illustrates photographs of the bladder taken after administering the composition for contrast imaging of cancer containing the compound III to the bladder of an MB49 mouse bladder cancer model, and washing the bladder with saline after 30 minutes (left: under white light, right: near-infrared fluorescence image).
[Fig. 4] Fig. 4 illustrates photographs of the bladder taken after administering the composition for contrast imaging of cancer containing the compound III to the bladder of a normal mouse, and washing the bladder with saline after 30 minutes (left: under white light, right: near-infrared fluorescence image).
[Fig. 5] Fig. 5 illustrates photographs of a cancer tissue taken out through observation with near-infrared fluorescence one day after tail vein administration of the composition for contrast imaging of cancer containing the compound III to an MB49 mouse bladder cancer model (left: under white light, right: near-infrared fluorescence image).

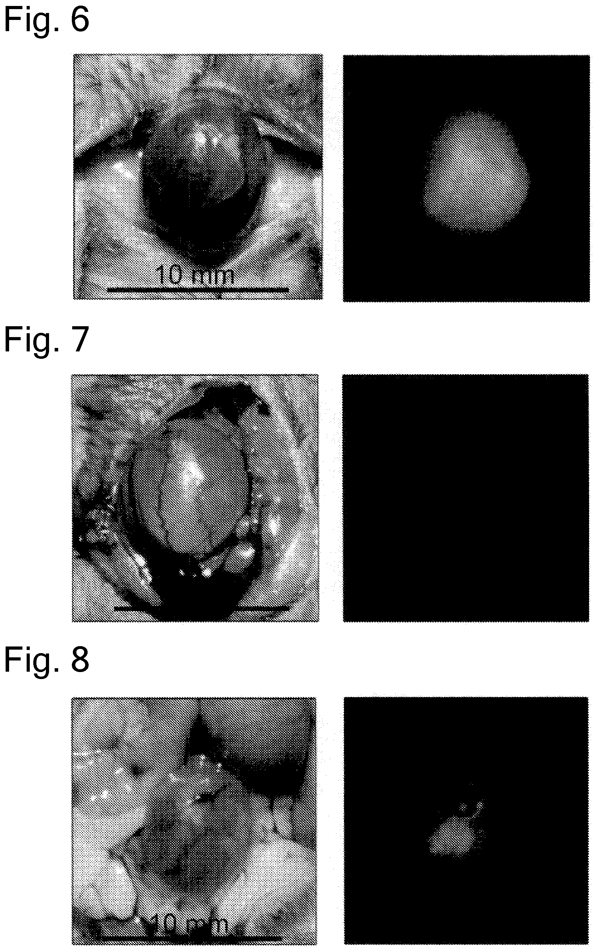

[Fig. 6] Fig. 6 illustrates photographs of the bladder taken one day after tail vein administration of a composition for contrast imaging of cancer containing a compound IV to an MB49 mouse bladder cancer model (left: under white light, right: near-infrared fluorescence image).
[Fig. 7] Fig. 7 illustrates photographs of the bladder taken one day after tail vein administration of the composition for contrast imaging of cancer containing the compound IV to a normal mouse (left: under white light, right: near-infrared fluorescence image).
[Fig. 8] Fig. 8 illustrates photographs of the bladder taken after administering the composition for contrast imaging of cancer containing the compound IV to the bladder of an MB49 mouse bladder cancer model, and washing the bladder with saline after 10 minutes (left: under white light, right: near-infrared fluorescence image).

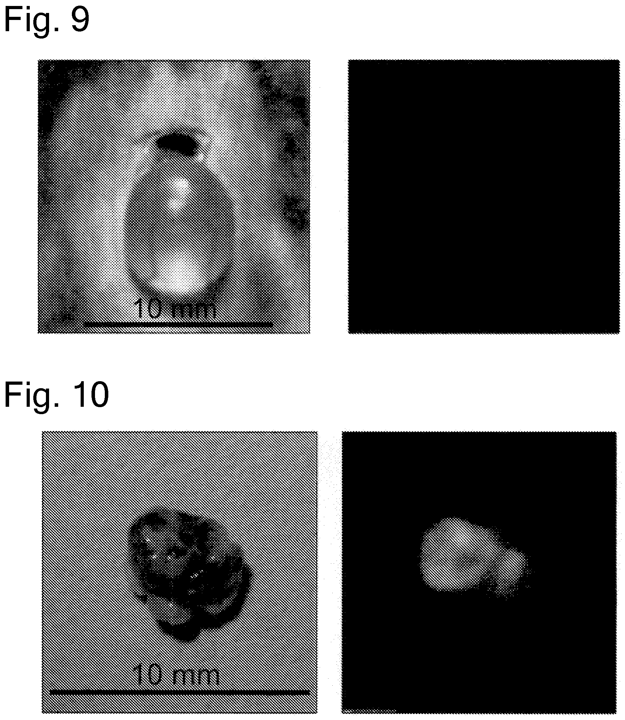

[Fig. 9] Fig. 9 illustrates photographs of the bladder taken after administering the composition for contrast imaging of cancer containing the compound IV to the bladder of a normal mouse, and washing the bladder with saline after 10 minutes (left: under white light, right: near-infrared fluorescence image).
[Fig. 10] Fig. 10 illustrates photographs of a cancer tissue taken out through observation with near-infrared fluorescence one day after tail vein administration of the composition for contrast imaging of cancer containing the compound IV to an MB49 mouse bladder cancer model (left: under white light, right: near-infrared fluorescence image).

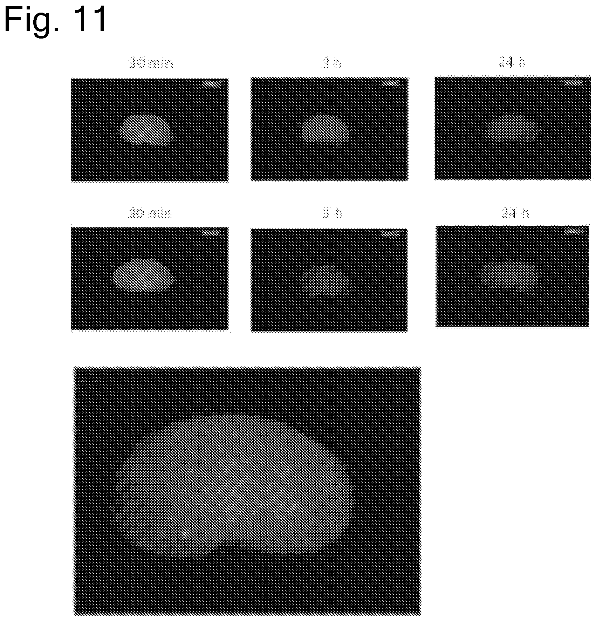

[Fig. 11] Fig. 11 illustrates near-infrared fluorescence macro images of the surface of the kidney of a normal rat (upper) and the surface of the kidney having pretumor/tumor induced with ferric nitrilotriacetate (lower) taken 30 minutes, 3 hours, and 24 hours after administering the composition for contrast imaging of cancer containing the compound III to a normal rat and a rat intraperitoneally administered with ferric nitrilotriacetate. The lowermost enlarged image is a near-infrared fluorescence image of the kidney having tumor induced with ferric nitrilotriacetate taken 3 hours after administering the composition for contrast imaging of cancer containing the compound III. Fluorescence brightness is adjusted for making the fluorescence image easily seen.

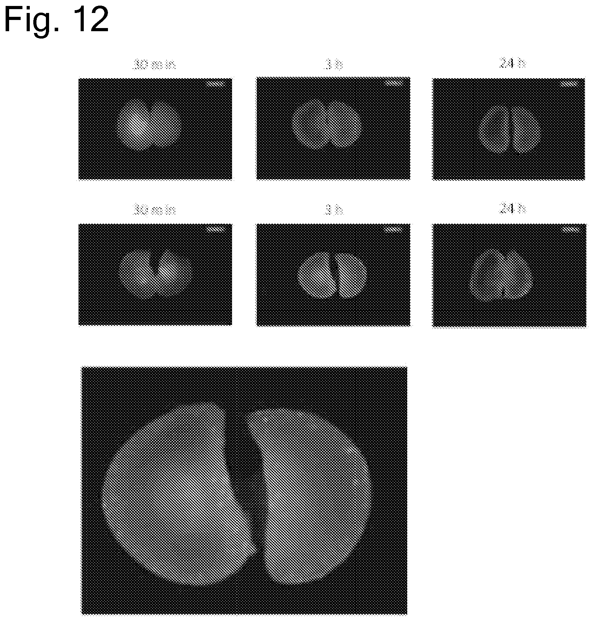

[Fig. 12] Fig. 12 illustrates near-infrared fluorescence macro images of a cross section of the kidney of a normal rat (upper) and a cross section of the kidney having pretumor/tumor induced with ferric nitrilotriacetate (lower) taken 30 minutes, 3 hours, and 24 hours after administering the composition for contrast imaging of cancer containing the compound III to a normal rat and a rat intraperitoneally administered with ferric nitrilotriacetate. The lowermost enlarged image is a near-infrared fluorescence image of the kidney having tumor induced with ferric nitrilotriacetate taken 3 hours after administering the composition for contrast imaging of cancer containing the compound III. Fluorescence brightness is adjusted for making the fluorescence image easily seen.

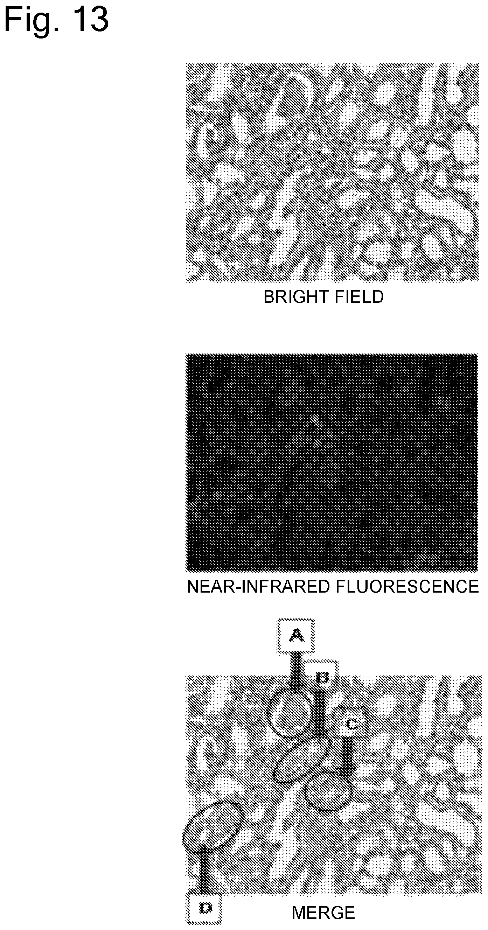

[Fig. 13] Fig. 13 illustrates near-infrared fluorescence micro images of a tissue section (renal cortex portion) of the kidney taken 3 hours after administering the composition for contrast imaging of cancer containing the compound III to a rat having pretumor/tumor induced with ferric nitrilotriacetate (upper: bright field, middle: near-infrared fluorescence, lower: merge). Fluorescence brightness is optionally adjusted for making the fluorescence image easily seen. Symbols used in the figures denote the following:
   A: column formed in the lumen of renal tubule (initial stage of renal cyst)
   B, C, D: distal renal tubule
[Fig. 14] Fig. 14 illustrates near-infrared fluorescence images taken after administering the composition for contrast imaging of cancer containing the compound III, or indocyanine green (ICG) into tumor of a 4T1 mouse syngeneic orthotopic breast cancer model (upper: after 30 minutes, middle: after 60 minutes, lower: after 3 hours).

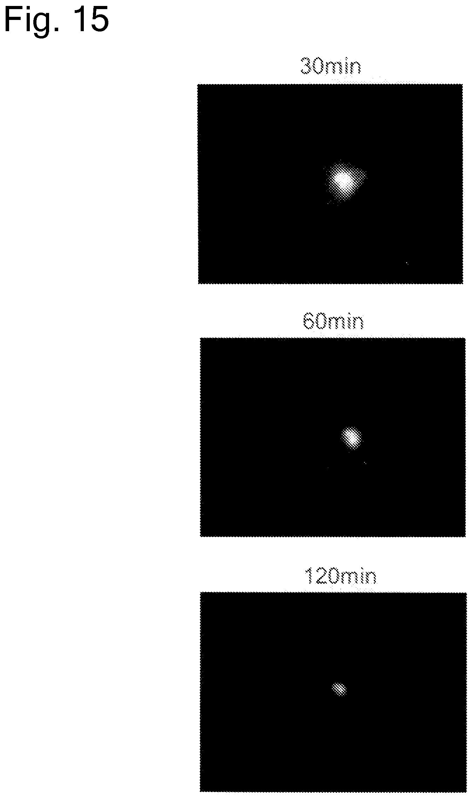

[Fig. 15] Fig. 15 illustrates near-infrared fluorescence images taken after administering the composition for contrast imaging of cancer containing the compound III into tumor of a CT26 mouse syngeneic colorectal cancer subcutaneous transplant model (upper: after 30 minutes, middle: after 60 minutes, lower: after 120 minutes).

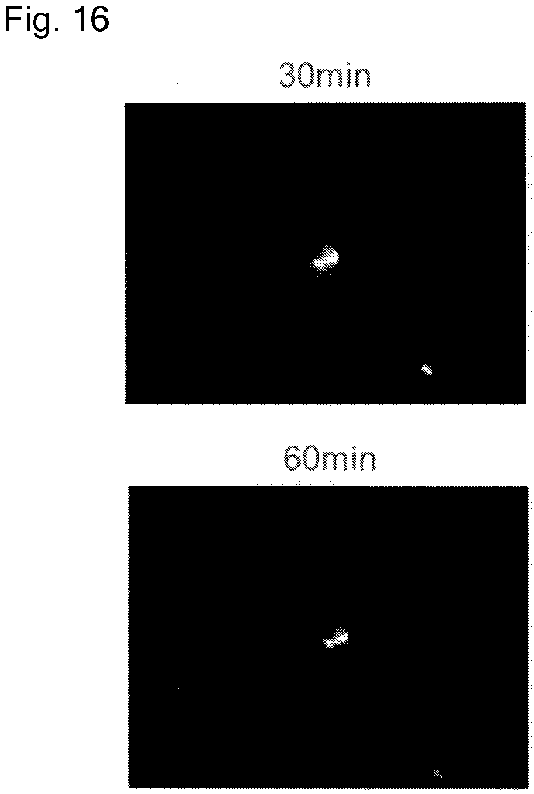

[Fig. 16] Fig. 16 illustrates near-infrared fluorescence images taken after administering the composition for contrast imaging of cancer containing the compound III into tumor of a B 16F 1 mouse syngeneic orthotopic melanoma model (upper: after 30 minutes, lower: after 60 minutes).

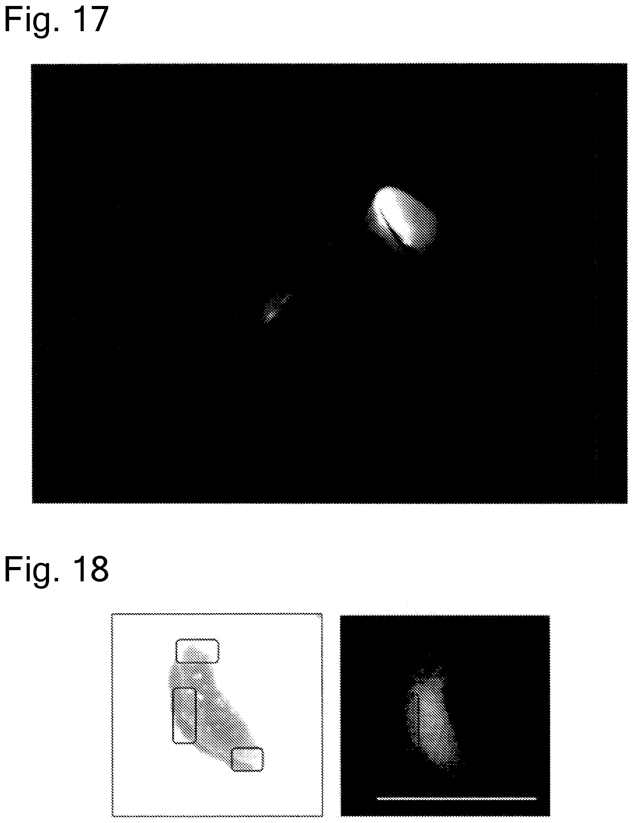

[Fig. 17] Fig. 17 is a near-infrared fluorescence image, taken by fluorescence imaging performed during 4T1 tumor excision operation, of a state of the tumor isolated from a mouse and picked up with tweezers.
[Fig. 18] Fig. 18 illustrates a color image (left) and a near-infrared fluorescence image (right) of a cross section of the lung taken 10 minutes after tail vein administration of the composition for contrast imaging of cancer containing the compound III to a lung cancer-bearing mouse. A white site in the near-infrared fluorescence image corresponds strong fluorescence, and a framed site corresponds to a cancer site (scale bar: 10 mm).

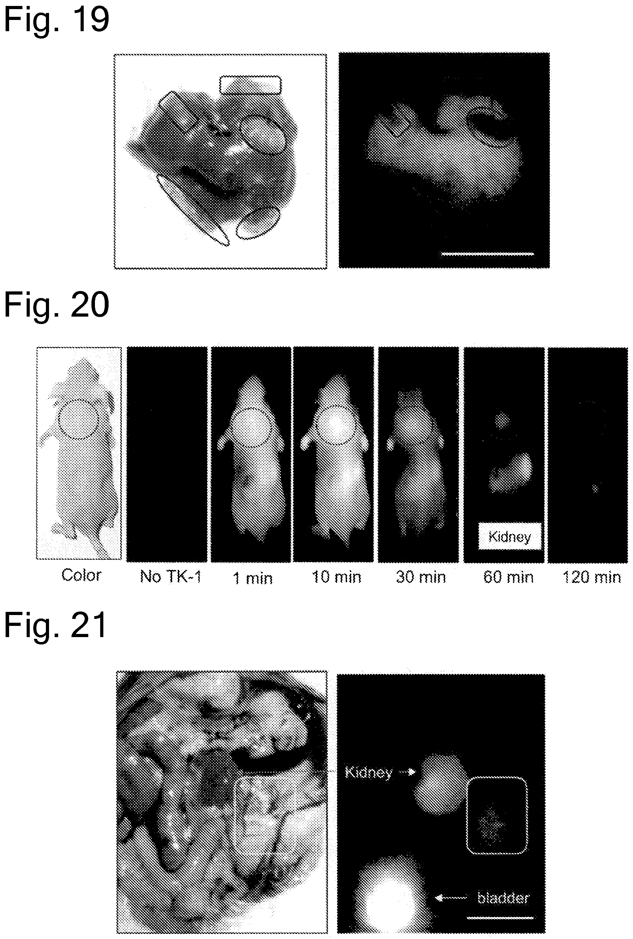

[Fig. 19] Fig. 19 illustrates a color image (left) and a near-infrared fluorescence image (right) of the liver taken after tail vein administration of the composition for contrast imaging of cancer containing the compound III to a liver cancer-bearing mouse. White in the near-infrared fluorescence image corresponds to fluorescence, and framed sites in the color image and the near-infrared fluorescence image correspond to liver cancer sites (scale bar: 10 mm).
[Fig. 20] Fig. 20 illustrates near-infrared fluorescence images taken over time after tail vein administration of the composition for contrast imaging of cancer containing the compound III to a human stomach cancer cell subcutaneous transplant mouse. White in the near-infrared fluorescence images corresponds to fluorescence. A circled site in the images corresponds to a cancer tissue.
[Fig. 21] Fig. 21 illustrates a color image (left) and a near-infrared fluorescence image (right) of the inside of the abdominal cavity taken after tail vein administration of the composition for contrast imaging of cancer containing the compound III to a peritoneal cancer mouse model. White in the near-infrared fluorescence image corresponds to fluorescence. Framed sites in the color image and the near-infrared fluorescence image correspond to peritoneal cancer (scale bar: 10 mm).

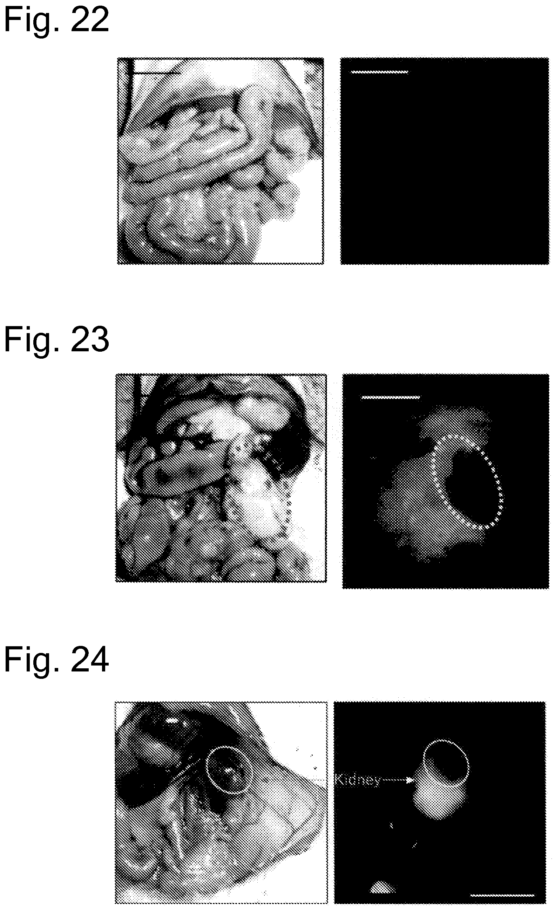

[Fig. 22] Fig. 22 illustrates a color image (left) and a near-infrared fluorescence image (right) of the inside of the abdominal cavity taken 10 minutes after tail vein administration of the composition for contrast imaging of cancer containing the compound III to a normal mouse. White in the near-infrared fluorescence image corresponds to fluorescence (scale bar: 10 mm).
[Fig. 23] Fig. 23 illustrates a color image (left) and a near-infrared fluorescence image (right) of the inside of the abdominal cavity taken 10 minutes after tail vein administration of the composition for contrast imaging of cancer containing the compound III to a peritoneal dissemination mouse model having human stomach cancer cell intraperitoneally transplanted. White in the near-infrared fluorescence image corresponds to fluorescence, and a site surrounded by a dotted line in the images corresponds to a visually recognized cancer solid tissue (scale bar: 10 mm).
[Fig. 24] Fig. 24 illustrates a color image (left) and a near-infrared fluorescence image (right) of the inside of the abdominal cavity taken 10 minutes after tail vein administration of the composition for contrast imaging of cancer containing the compound III to a kidney cancer mouse. White in the near-infrared fluorescence image corresponds to fluorescence, and a framed site in the images corresponds to a visually recognized cancer tissue (scale bar: 10 mm).

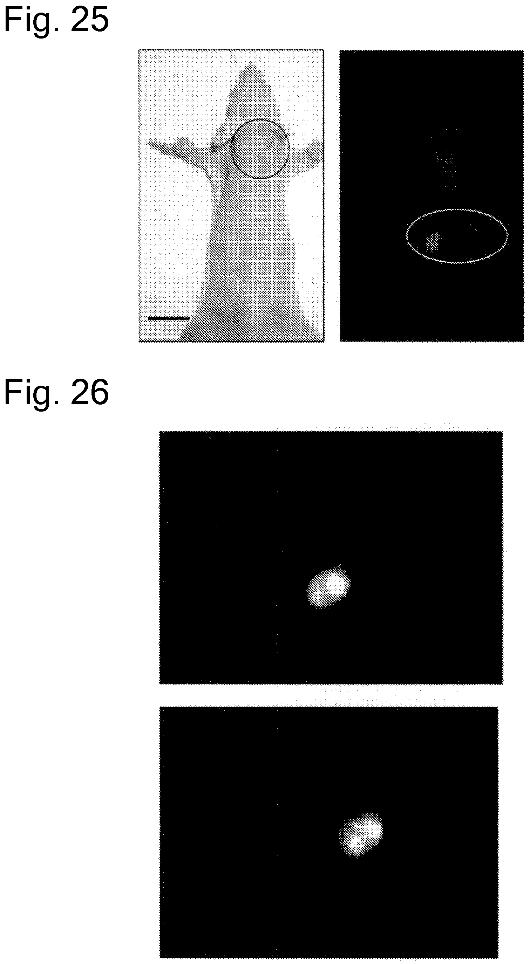

[Fig. 25] Fig. 25 illustrates a color image (left) and a near-infrared fluorescence image (right) taken 30 seconds after tail vein administration of the composition for contrast imaging of cancer containing the compound III to a human esophageal cancer subcutaneous transplant mouse model. A circled site corresponds to a cancer tissue (scale bar: 10 mm).
   Fluorescence in an elliptical frame corresponds to near-infrared fluorescence from the right and left kidneys, and white in the near-infrared fluorescence image corresponds to fluorescence.
[Fig. 26] Fig. 26 illustrates near-infrared fluorescence images taken after administering the composition for contrast imaging of cancer containing the compound III into tumor of a human tongue cancer subcutaneous transplant model (upper: immediately after administration, lower: after 50 minutes).

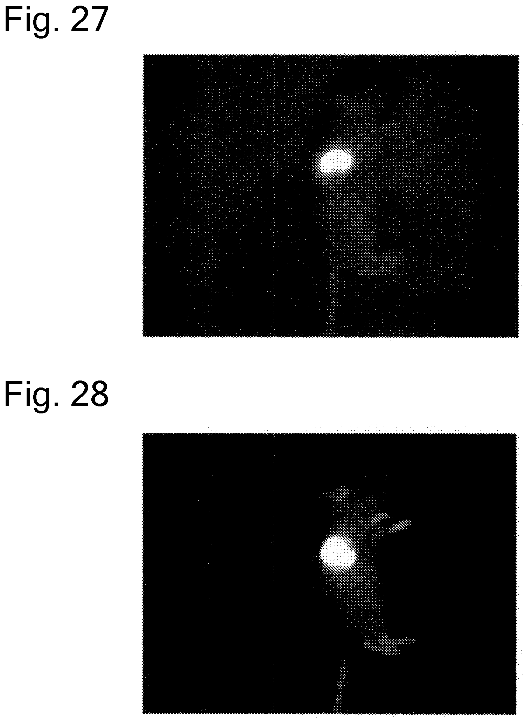

[Fig. 27] Fig. 27 illustrates near-infrared fluorescence images taken after administering the composition for contrast imaging of cancer containing the compound III into tumor of a human neuroblastoma subcutaneous transplant model (1 hour after the administration).
[Fig. 28] Fig. 28 illustrates near-infrared fluorescence images taken after administering the composition for contrast imaging of cancer containing the compound III into tumor of a human glioblastoma subcutaneous transplant model (1 hour after the administration).

### DESCRIPTION OF EMBODIMENTS

The present invention will now be described in detail, and it is noted that the present invention is not limited to the following aspects.

In one aspect, the present invention provides a composition for contrast imaging of cancer, and the present invention provides a pharmaceutical composition for contrast imaging of cancer used in the pharmaceutical field. A composition for contrast imaging of cancer of the present invention contains a cyclodextrin-bonded indocyanine compound represented by the following formula, or a pharmaceutically acceptable salt thereof (hereinafter, also simply referred to as the compound A): 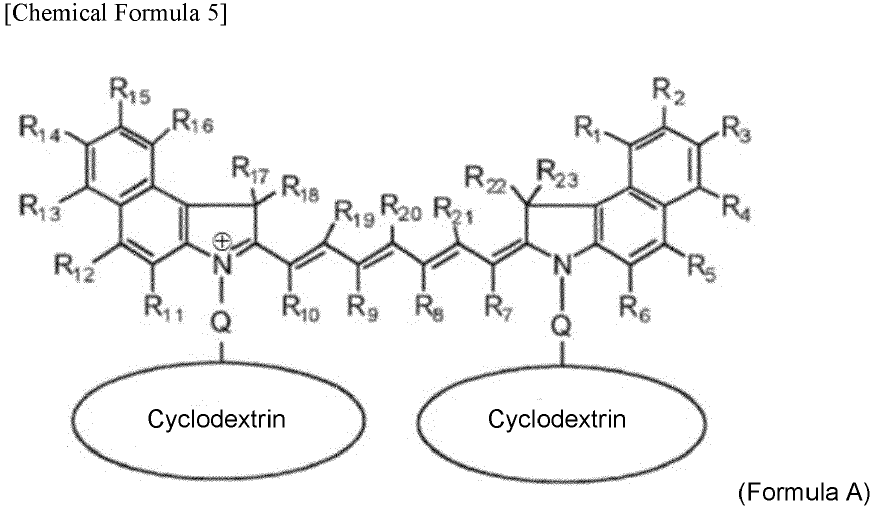 In formula A, two Qs are each capable of being independently selected, are optionally the same or different, and are each * 1-(CH₂)a-CO-NH-(CH₂)b-*3, wherein a is an integer of 2 or more and 6 or less, b is an integer of 2 or more and 6 or less, * 1 is N of the indocyanine skeleton, and *3 is a bonding portion formed by replacing any one of three OH groups of any D-glucose contained in cyclodextrin with -O-; R1 to R23 each independently are a hydrogen atom, an alkyl group, an aryl group, a halogen atom, an alkoxyl group, an amino group, a carboxyl group, a formyl group, a sulfonyl group, a sulfonic acid group, a phosphoric acid group, an alkyloxycarbonyl group, an aryloxycarbonyl group, an alkylcarbonyl group, an arylcarbonyl group, or a heterocycle, R1 to R23 are each optionally independently unsubstituted, or substituted with a substituent, when the substituent is carboxylic acid, sulfonic acid, or phosphoric acid, a hydrogen ion is optionally dissociated from the substituent to be replaced with a metal ion; R8 and R9 optionally together form a cyclic structure of CH₂, CH₂CH₂, CH₂CH₂CH₂, or CH₂CH₂CH₂CH₂, and one or more hydrogen atoms of the cyclic structure are optionally replaced with an aryl group, a halogen atom, an alkoxy group, an amino group, a carboxyl group, a formyl group, a sulfonyl group, a sulfonic acid group, a phosphoric acid group, an alkyloxycarbonyl group, an aryloxycarbonyl group, an alkylcarbonyl group, an arylcarbonyl group, or a heterocycle. In one aspect, the numbers of carbon atoms of R1 to R23 in formula A are each independently 1 to 5, and may be 1 to 3. Here, when R1 to R23 are substituted with carboxylic acid, sulfonic acid, or phosphoric acid, a hydrogen ion of the carboxylic acid, sulfonic acid, or phosphoric acid is optionally replaced with sodium, potassium, or magnesium.

An "alkyl group" refers to a linear or branched alkyl group optionally having a substituent, and having 1 to 20 carbon atoms, and examples include linear groups and groups bonded in a branched manner, such as methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, and icosanyl.

An "aryl group" can be an aromatic hydrocarbon having 6 to 20 carbon atoms such as phenyl or naphthyl.

In the present invention, examples of an "alkoxyl group" include alkoxyl groups having 1 to 20 carbon atoms bonded in a linear manner or a branched manner, or the like, such as methoxy, ethoxy, propoxy, butoxy, pentyloxy, hexyloxy, methoxyethoxy, methoxypropoxy, ethoxyethoxy, ethoxypropoxy, methoxyethoxyethoxy groups.

An "alkyloxycarbonyl group" refers to a group represented by alkyl group-O-C(=O)-. An "aryloxycarbonyl group" refers to a group represented by aryl group-O-C(=O)-. An "alkylcarbonyl group" refers to a group represented by alkyl group-C(=O)-. An "arylcarbonyl group" refers to a group represented by aryl group-C(=O)-.

A "heterocycle" means a 5- to 7-membered unsaturated heterocycle or saturated heterocycle containing 1 to 4 hetero atoms selected from a nitrogen atom, an oxygen atom, or a sulfur atom, or a bicyclic heterocycle condensed with a benzene ring or another heterocycle. Examples of the aromatic heterocycle include pyrrole, imidazole, pyrazole, pyridine, pyrazine, pyrimidine, pyridazine, triazole, thiophen, thiopyran, furan, pyran, dioxolane, thiazole, isothiazole, thiadiazole, thiazine, oxazole, isoxazole, oxadiazole, dioxazole, oxazine, oxadiazine, dioxazine, or the like. Examples of the saturated heterocycle include pyrrolidine, piperidine, piperazine, morpholine, thiomorpholine, or the like. Examples of a condensed aromatic heterocycle include indole, isoindole, indazole, quinoline, quinazoline, quinoxaline, isoquinoline, benzimidazole, benzothiophene, benzothiazole, benzofuran, benzofurazan, imidazopyridine, imidazopyrazine, pyridopyridine, phthalazine, naphthyridine, indridine, purine, quinolizine, cinnoline, isocoumarin, chroman, or the like.

A "halogen atom" means a fluoro, chloro, bromo, or iodo atom. In one aspect, it is a fluoro, chloro, or bromo atom, and in another aspect, it is a chloro atom.

There may exist 1 to 3 "substituents" the same as or different from each other. A "substituent on an amino group in secondary, tertiary, or quaternary one" refers to a halogen atom, an alkyl group, or the like.

The cyclodextrin-bonded indocyanine compound that is an active ingredient of the present composition is a cyclodextrin-bonded indocyanine compound obtained by covalently bonding an indocyanine and cyclic sugar chain cyclodextrin, and can be in a state where at least a part of a naphthyl group of indocyanine is included in the cavity of cyclodextrin. Besides, if a naphthyl group of indocyanine is included in the cavity of cyclodextrin, and near-infrared fluorescence is emitted, an indocyanine group may have a substituent.

In order that at least a part of a naphthyl group of indocyanine is included in the cavity of cyclodextrin, a spacer of Q may be used so as to covalently bond an indocyanine to cyclic sugar chain cyclodextrin via the spacer. Here, when the length of the spacer is adjusted, the extent of inclusion of the naphthyl group of indocyanine in the cavity of cyclodextrin can be controlled. Considering easiness of the inclusion in cyclodextrin, the space of Q may have 7 or more and 9 or less atoms in a structure between a nitrogen atom in the structure corresponding to indocyanine and an oxygen atom in the structure corresponding to cyclodextrin.

Cyclodextrin in formula A is not especially limited, and examples include α-cyclodextrin, β-cyclodextrin, and γ-cyclodextrin, and the cyclodextrin may be provided with a substituent. In one aspect, the cyclodextrin in formula A is β-cyclodextrin. Besides, the cyclodextrin may be provided with a substituent.

In the compound used in the composition for contrast imaging of cancer of the present invention, at least a part of a naphthyl moiety of indocyanine can be included in cyclodextrin in a molecule. The compound of the present invention is in an isomerization equilibrium state in an aqueous solution, and can be in an equilibrium state between inclusion type and non-inclusion type.

The compound of the present invention emits fluorescence in a near-infrared region (700 nm to 2500 nm), for example, in the vicinity of 800 to 830 nm, and when administered to a living body to obtain a near-infrared fluorescence image therefrom, an abnormal area of living tissues can be extracted on a micro level and a macro level.

In one aspect, the cyclodextrin-bonded indocyanine compound of the present invention is a compound represented by the following formula, or a pharmaceutically acceptable salt thereof (hereinafter, also simply referred to as the compound B): 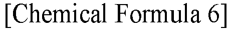 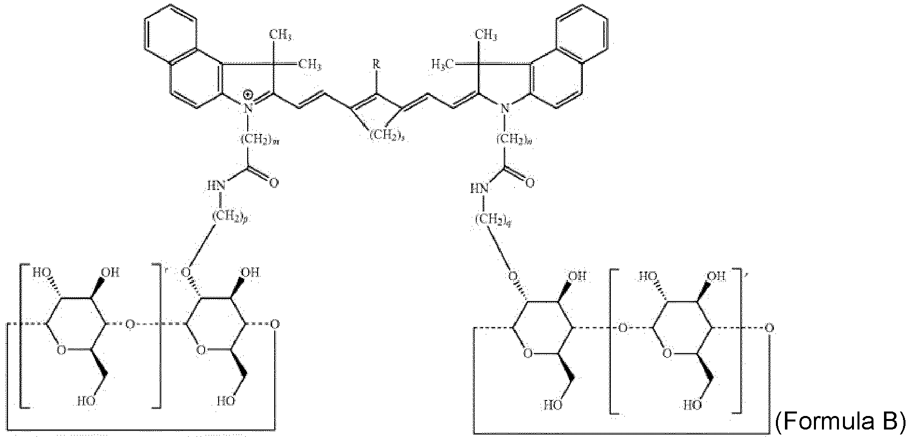 In formula B, m, n, p, and q are each independently an integer of 2 or more and 6 or less, r is an integer of 5 or more and 7 or less, s is an integer of 0 or more and 4 or less, and R is a hydrogen atom, an alkyl group, an aryl group, a halogen atom, an alkoxy group, an amino group, a carboxyl group, a formyl group, a sulfonyl group, a sulfonic acid group, an alkyloxycarbonyl group, an aryloxycarbonyl group, an alkylcarbonyl group, an arylcarbonyl group, or a heterocycle. In one aspect, R in formula B has 1 to 5 carbon atoms, and may have 1 to 3 carbon atoms.

In one aspect, the cyclodextrin-bonded indocyanine compound of the present invention is a compound represented by the following formula, or a pharmaceutically acceptable salt thereof (hereinafter, also simply referred to as the compound C): 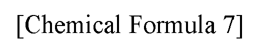 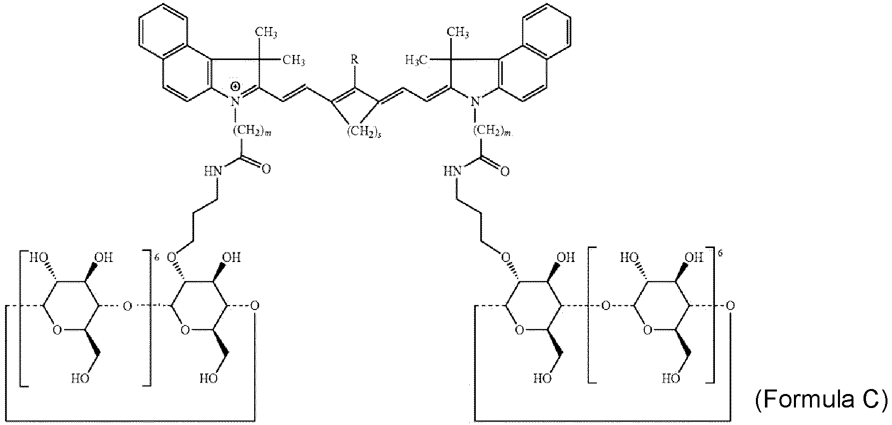 In formula C, m and n are each independently an integer of 2 or more and 6 or less, s is an integer of 0 or more and 4 or less, and R is a hydrogen atom, an alkyl group, an aryl group, a halogen atom, an alkoxy group, an amino group, a carboxyl group, a formyl group, a sulfonyl group, a sulfonic acid group, an alkyloxycarbonyl group, an aryloxycarbonyl group, an alkylcarbonyl group, an arylcarbonyl group, or a heterocycle. In one aspect, R in formula C has 1 to 5 carbon atoms, and may have 1 to 3 carbon atoms.

In one aspect, in the compound of the present invention or a pharmaceutically acceptable salt thereof, R in formula B or formula C is an alkoxy group.

In one aspect, the composition for contrast imaging of cancer of the present invention contains a compound represented by formula I, or a pharmaceutically acceptable salt thereof (hereinafter, also simply referred to as the compound I): 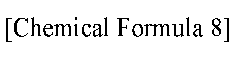 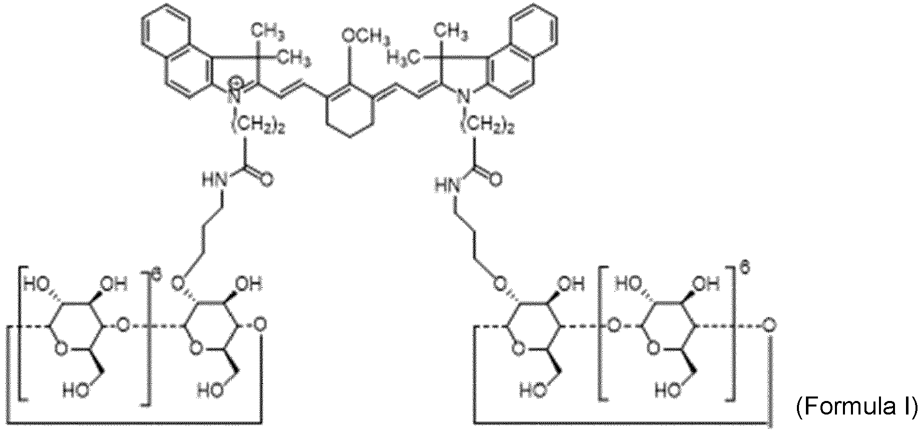 3-(3-{[3-(cyclomaltoheptaose-21-O-yl)propyl]amino}-3-oxopropyl)-2-{(1E)-2-[(3E)-3-{(2E)-2-[3-(3-{[3-(cyclomaltoheptaose-21-O-yl)propyl]amino}-3-oxopropyl)-1,1-dimethyl-1,3-dihydro-2H-b enzo[e]indol-2-ylidene] ethylidene}-2-methoxycyclohexa-1-en-1-yl]ethene-1-yl}-1,1-dimethyl-1H-benzo[e]indol-3-ium

In one aspect of the present invention, the composition for contrast imaging of cancer of the present invention contains a compound represented by formula II, or a pharmaceutically acceptable salt thereof (hereinafter, also simply referred to as the compound II):

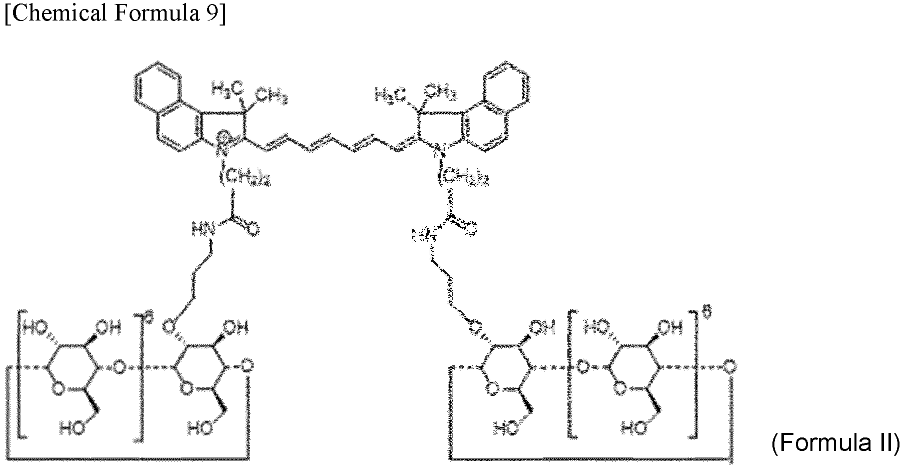

In one aspect, the composition for contrast imaging of cancer of the present invention is for systemic administration.

In one aspect, the composition for contrast imaging of cancer of the present invention is a composition for contrast imaging of bladder cancer.

In one aspect, the composition for contrast imaging of cancer of the present invention is a composition for contrast imaging of kidney cancer.

In one aspect, the composition for contrast imaging of cancer of the present invention is a composition for contrast imaging of lung cancer.

In one aspect, the composition for contrast imaging of cancer of the present invention is a composition for contrast imaging of liver cancer.

In one aspect, the composition for contrast imaging of cancer of the present invention is a composition for contrast imaging of stomach cancer.

In one aspect, the composition for contrast imaging of cancer of the present invention is a composition for contrast imaging of peritoneal cancer.

In one aspect, the composition for contrast imaging of cancer of the present invention is a composition for contrast imaging of peritoneal dissemination.

In one aspect, the composition for contrast imaging of cancer of the present invention is a composition for contrast imaging of esophageal cancer.

In one aspect, the composition for contrast imaging of cancer of the present invention is for intravesical administration via the urethra.

In one aspect, the composition for contrast imaging of cancer of the present invention is for topical administration.

In one aspect, the composition for contrast imaging of cancer of the present invention is a composition for contrast imaging of breast cancer.

In one aspect, the composition for contrast imaging of cancer of the present invention is a composition for contrast imaging of colorectal cancer.

In one aspect, the composition for contrast imaging of cancer of the present invention is a composition for contrast imaging of skin cancer.

In one aspect, the composition for contrast imaging of cancer of the present invention is a composition for contrast imaging of head and neck cancer.

In one aspect, the composition for contrast imaging of cancer of the present invention is a composition for contrast imaging of brain cancer.

In one aspect of the present invention, the composition for contrast imaging of cancer of the present invention contains a chloride of the compound I (compound III).

In one aspect of the present invention, the composition for contrast imaging of cancer of the present invention contains a chloride of the compound II (compound IV).

One aspect of the present invention is use of the compound A, B, C, I, II, III, or IV for producing the composition for contrast imaging of cancer of the present invention.

One aspect of the present invention is use of the compound A, B, C, I, II, III, or IV for contrast imaging of cancer.

In one aspect of the present invention, the composition for contrast imaging of cancer of the present invention is an injection liquid.

The compound of the present invention or a pharmaceutically acceptable salt thereof can be obtained by, for example, a method described in U.S. Patent Publication No. 2012/0302881, or the like.

A "pharmaceutically acceptable salt" means an acid addition salt of the compound, and can be obtained by ordinary salt forming reaction. Specific examples include acid addition salts with inorganic acids such as hydrochloric acid, hydrobromic acid, hydroiodic acid, sulfuric acid, nitric acid, and phosphoric acid, and with organic acids such as formic acid, acetic acid, propionic acid, oxalic acid, malonic acid, succinic acid, fumaric acid, maleic acid, lactic acid, malic acid, mandelic acid, tartaric acid, dibenzoyl tartaric acid, di-toluoyl tartaric acid, citric acid, methanesulfonic acid, ethanesulfonic acid, benzenesulfonic acid, p-toluenesulfonic acid, aspartic acid, glutamic acid.

The composition for contrast imaging of cancer of the present invention contains the compound of the present invention or a pharmaceutically acceptable salt thereof, and can contain a pharmaceutically acceptable excipient or the like. The composition for contrast imaging of cancer of the present invention can be prepared by a usually employed method using a pharmaceutically acceptable excipient or carrier, or the like.

The composition for contrast imaging of cancer of the present invention is not especially limited in the administration route, and may be systemically administered or topically administered, or parenterally administered or orally administered. The administration route may be appropriately selected in accordance with an administration subject or cancer tissue, or the like, and for example, when it is intravesically administered via the urethra, bladder cancer can be imaged. The composition for contrast imaging of cancer of the present invention can be administered to a subject by, for example, intravenous systemic administration, or intraarticular, intramuscular, or intra-cancer tissue topical administration, or the like. For example, the composition for contrast imaging of cancer of the present invention is not especially limited in the dosage form, and in one aspect, the composition is a liquid. To the composition for contrast imaging of cancer of the present invention, a pharmaceutically acceptable additive such as a suspending agent, a dissolution assisting agent, a tonicity agent, a preservative, an adsorption inhibitor, a soothing agent, a sulfur-containing reducing agent, or an antioxidant can be added, if necessary, in addition to a pharmaceutically acceptable excipient or carrier in accordance with the dosage form.

An injection agent for parenteral administration contains, for example, an aseptic aqueous or non-aqueous solution agent, suspension agent, or emulsion agent. Examples of an aqueous solvent include distilled water for injection and saline. Examples of a non-aqueous solvent include alcohols such as ethanol. Such a composition may further contain a tonicity agent, a preservative, a wetting agent, an emulsifier, a dispersant, a stabilizer, or a dissolution assisting agent. These are sterilized by, for example, filtration through a bacteria retention filter, addition of a bactericide, or irradiation. Besides, these may be used to produce an aseptic solid composition to be dissolved or suspended in aseptic water or an aseptic solvent for injection before use.

A dose may be appropriately determined in accordance with individual case in consideration of the symptom, age, sex, tumor size and the like of the administration subject. In intravenous administration, a daily dose is, for example, about 0.0001 to 10 mg/kg body weight once a day or dividedly a plurality of times a day in a preferable aspect. Alternatively, in topical administration to cancer, for example, a dose of about 0.001 to 100 mg/kg body weight can be administered once or dividedly a plurality of times.

Although varied depending on the administration route, the dosage form, the administration site, and the types of an excipient and an additive, the composition for contrast imaging of the present invention may contain, as an active ingredient, the compound of the present invention in an amount of 0.01 to 99% by weight, and in one aspect, may contain the compound of the present invention in an amount of 0.01 to 50% by weight.

The pharmaceutical composition for contrast imaging of cancer of the present invention is advantageously used as a pharmaceutical composition for contrast imaging of cancer for systemic administration in some cases, and is advantageously used as a pharmaceutical composition for contrast imaging of cancer for topical administration in other cases depending on the type of cancer.

In one aspect of administration time, the administration is pre-operative administration or intraoperative administration. In one aspect of administration time, the administration is intraoperative administration.

In one aspect, the present invention provides a cancer imaging method. In the present invention, when an effective amount of a composition for contrast imaging of cancer is administered to a subject, and desired cancer is irradiated with near-infrared light with a near-infrared fluorescence imaging device to detect near-infrared fluorescence emitted from the compound of the present invention, the desired cancer can be imaged.

Cancer to be imaged by the present invention is not especially limited, and for example, cancers in the kidney, the bladder, the ureter, the urethra, the esophagus, the stomach, the small intestine, the large intestine, the mammary gland, the skin, the liver, the lung, the peritoneum, the head and neck, the brain, and the like can be imaged. In other words, kidney cancer, bladder cancer, esophageal cancer, stomach cancer, colorectal cancer, breast cancer, skin cancer, lung cancer, liver cancer, peritoneal cancer, peritoneal dissemination, head and neck cancer, brain cancer and the like can be imaged with near-infrared light by the present invention. It is noted that these cancers encompass cancer caused in the organ due to metastasis of cancer originating from another organ. For example, lung cancer encompasses cancer originating from kidney cancer. Here, brain cancer means malignant tumor (cancer) caused in the brain, and examples include neuroblastoma, and glioblastoma. Besides, head and neck cancer means malignant tumor (cancer) caused in the head and neck, and an example includes tongue cancer.

Through systemic administration, for example, bladder cancer, kidney cancer, lung cancer, liver cancer, stomach cancer, peritoneal cancer, peritoneal dissemination, and esophageal cancer can be imaged.

Through topical administration, for example, breast cancer, colorectal cancer, skin cancer, head and neck cancer, and brain cancer can be imaged.

In one aspect, the cancer is a primary cancer initially caused in the organ.

In one aspect, the cancer is a metastatic cancer caused in the organ through metastasis of cancer originating from another organ.

A representative example of an imaging method at the experimental level is as follows:
1. Creation of Cancer-bearing Mouse
   Referring, for example, to "Gan - Shikkan Model no Sakusei to Riyo (Cancer - Creation and Use of Disease Model)" (Takuro Nakamura, LIC, 2012), a cancer-bearing mouse is created by an ordinary method.
2. Case of Bladder Cancer
   An saline of the compound of the present invention is intravenously administered to a mouse, and after about 1 day, the bladder is irradiated with near-infrared light with a near-infrared fluorescence imaging device to detect near-infrared fluorescence emitted from the compound. The dose of the compound is, for example, 0.01 to 10 mg per kg of the mouse, and in another aspect, is 0.1 to 1 mg.
3. Case of Bladder Cancer
   A saline of the compound of the present invention is administered to the bladder via the urethra of the mouse, after about 10 minutes to 2 hours, the inside of the bladder is washed with water or saline, and the bladder is irradiated with near-infrared light with a near-infrared fluorescence imaging device to detect near-infrared fluorescence. The saline containing the compound of the present invention is used in a volume capable of filling the bladder of the subject. The saline containing the compound of the present invention contains the compound of the present invention in a concentration of, for example, 0.00001 to 1 mg/mL, and in another aspect, in a concentration of 0.001 to 0.1 mg/mL. The number of times of washing the inside of the bladder can be changed in accordance with the concentration of the compound injected. For washing the inside of the bladder, water or saline is filled/discharged in/from the entire bladder, and the liquid in the bladder is exchanged 5 times to 10 times.
4. Case of Another Cancer
   0.05 to 0.5 mL of a saline of the compound of the present invention is topically administered to cancer of a cancer-bearing mouse, and thereafter, the desired cancer is irradiated with near-infrared light with a near-infrared fluorescence imaging device to detect near-infrared fluorescence emitted from the compound. The saline containing the compound of the present invention contains the compound of the present invention in a concentration of, for example, 0.00001 to 1 mg/mL.

30 seconds to 120 minutes after intravenous administration of a saline of the compound of the present invention to a mouse, near-infrared irradiation is performed with a near-infrared fluorescence imaging device on the body surface from outside the skin, or on the abdominal cavity with the abdomen incised, or on the thoracic cavity with the chest incised, or on excised organ desired to be observed, and then, near-infrared fluorescence emitted from the compound is detected. The dose of the compound is, for example, 0.00001 to 10 mg per kg of the weight, and in another aspect, is 0.01 to 1 mg.

Besides, the following aspects can be other aspects of the present invention.

In one aspect, the present invention provides a technique for discriminating a cancer tissue, and encompasses a method and a device for discriminating a cancer tissue. A cancer tissue can be accurately discriminated by the present invention, and hence, the present invention can be applied as a guide technique in surgery and the like. In one aspect of the present invention, the present invention enables objective resection not depending on judgement of an operator for specifying a cancer site.

In one aspect, the present invention provides a surgery support device, and is applicable to various surgeries. The device of the present invention may include an imaging device, and automated robotic resection is expected to be realized by, for example, determination of a resection site by artificial intelligence (AI) or the like using a computer or the like.

In one aspect, the present invention provides use of the compound of the present invention for producing a composition for contrast imaging of cancer. In another aspect, the present invention provides the compound of the present invention for producing a composition for contrast imaging of cancer. In still another aspect, the present invention provides use of the compound of the present invention for imaging cancer.

The device of the present invention can be equipped with excitation light irradiation means and fluorescence intensity measurement means for a subject to which the compound of the present invention has been administered.

The excitation light irradiation means is means for irradiating the administered compound of the present invention with excitation light of a wavelength capable of generating fluorescence. The wavelength of the irradiated excitation light can be limited to an adequate range. When the wavelength is limited to a range as narrow as possible, fluorescence can be definitely separated from the excitation light. For limiting the wavelength, a light source emitting light of an adequate wavelength can be used, or the wavelength can be limited with a filter.

The aspect of the excitation light irradiation is not especially limited as long as the generated fluorescence can be measured with the fluorescence intensity measurement means described below. Examples of the excitation light include continuous light, pulsed light, and light having intensity changed or the like. When the intensity is to be changed, the intensity of the excitation light can be modulated by, for example, irradiation with a pulse of the excitation light at prescribed intervals or the like. For modulating the excitation light, it is preferable to modulate the intensity of the excitation light by employing pulse-amplitude modulation.

The excitation light irradiates a site to be irradiated using an adequate optical system. The site to be irradiated is a site where a cancer tissue is suspected to be present in a living body. An irradiation range of the excitation light is not especially limited, and the irradiation range is determined as necessary. For example, when a narrow range is irradiated, precise measurement can be performed in the irradiated narrow portion.

Besides, irradiation with the excitation light with the excitation light irradiation means is performed preferably with the influence of ambient light suppressed. For example, irradiation with the excitation light is performed preferably in a dark place, or with a portion to be irradiated with the excitation light covered from outside light.

The fluorescence intensity measurement means is means for measuring the intensity of fluorescence emitted from a portion that is irradiated with the excitation light by the excitation light irradiation means. The measurement of the fluorescence intensity is preferably performed through a filter capable of selectively transmitting the fluorescence emitted so as to exclude light other than the fluorescence (such as ambient light, and the excitation light).

When means for irradiation with excitation light having modulated intensity is used as the excitation light irradiation means, a component exhibiting change corresponding to the modulation can be separated from the measured intensity of the light as the fluorescence intensity. For example, when the intensity of the excitation light is modulated by pulse-amplitude modulation, the fluorescence intensity can be separated by demodulating a component of light changing in accordance with the modulated pulse intensity, and measuring the intensity. Therefore, the influence of ambient light on the measurement result of the fluorescence intensity can be reduced.

The device of the present invention may be equipped with fluorescence imaging means or shape imaging means, and besides, may be equipped with display means. According to the present invention, a site where a cancer tissue is present can be accurately identified, and therefore, the device of the present invention can be suitably used as a guiding device in surgery.

The fluorescence imaging means is means for obtaining distribution state data of the present compound in a living body by obtaining the intensity of the fluorescence emitted from the compound of the present invention excited by the excitation light irradiation means. In other words, this means is means for obtaining, in a part of the living body, distribution state data corresponding to the state of the distribution of the present compound as image data.

The fluorescence imaging means of the present invention can be constituted by, for example, a combination of an adequate optical system, and an imaging device such as a CCD. The resolution of the data to be obtained is set to a necessary value according to purpose. The measurement of the fluorescence intensity is preferably performed through a filter capable of selectively transmitting the fluorescence emitted so as to exclude light other than the fluorescence (such as ambient light, and the excitation light).

The shape imaging means is means for obtaining shape data of a part of the living body by obtaining the intensity of light of a wavelength excluding the fluorescence wavelength emitted from the compound of the present invention. In other words, this means is means for obtaining the shape data corresponding to the shape of a part of the living body as image data.

The shape imaging means can be constituted by an adequate optical system and an imaging device such as a CCD. The resolution of the data to be obtained is set to a necessary value according to purpose. In this case, the fluorescence emitted from the excitation light is not detected (or detection sensitivity thereof is set to be low). The shape imaging means shares, with the fluorescence imaging means described above, most of the optical system, and can employ a structure in which light of the wavelength corresponding to the fluorescence is guided to the fluorescence imaging means, and light of the other wavelength is guided to the shape imaging means by using a spectroscopic prism or the like in an optical path prior to final introduction into the imaging device. The spectroscopic prism can adequately control the wavelength of light to be separated by adequately forming a dichroic film and the like.

In the present invention, the fluorescence imaging means and the shape imaging means can be shared by one imaging device. In other words, the separation between the fluorescence and the other light may be performed mathematically after obtaining image data. Besides, the distribution state data can be obtained as a plurality of image data of a part of the living body from the surface in the depth direction. When the focus of the optical system employed in the fluorescence imaging means is moved in the depth direction, a plurality of image data in the depth direction can be obtained. Alternatively, when means capable of optically changing a focal length is employed as the excitation light irradiation means, and the focus is moved not on the surface of a part of the living body but inward in the depth direction, or the excitation light is narrowed to irradiate a part of the living body, fluorescence can be generated selectively not only on the surface of the living body but also inside in the depth direction of the living body, or the like, and thus, a circulating state in that portion can be visualized.

The display means is means for displaying the distribution state of the present compound in a part of the living body by, for example, displaying the shape data obtained by the shape imaging means to be superimposed on the distribution state data obtained by the fluorescence imaging means. When the wavelength of the fluorescence is out of the range of visible light, the wavelength of the fluorescence is converted into an adequate wavelength of visible light for displaying. It is preferable to display the distribution state data priorly to the shape data from the viewpoint of visualization of circulation. In particular, a portion where the amount of distribution of the present compound (namely, the fluorescence intensity) is low (whether or not it is low is determined according to the purpose of visualizing the circulation. For example, when the purpose is for visualizing a cancer tissue, it is a portion not circulating, namely, a portion having no fluorescence) is displayed discriminably from the other portion. For example, the portion can be displayed in a color different from the other portion, or can be displayed to be blinked. The superposition of the distribution state data and the shape data can be realized by logic on a computer or the like. The display of the data can be realized with a general display device. When this display device is installed between the living body and a measurer, the living body can be treated with the display device viewed.

The imaging encompasses positive imaging and negative imaging. Positive imaging means that the cyclodextrin-bonded indocyanine compound of the present invention is retained in cancer, and near-infrared fluorescence of the compound emitted in the cancer tissue is stronger than near-infrared fluorescence of the compound from a surrounding normal tissue, and hence the cancer tissue can be imaged discriminately from the surrounding normal tissue. Negative imaging means that the cyclodextrin-bonded indocyanine compound of the present invention is less retained in cancer as compared with a surrounding normal tissue, and near-infrared fluorescence of the compound emitted in the cancer tissue is weaker than near-infrared fluorescence of the compound from the surrounding normal tissue, and hence the cancer tissue can be imaged discriminately from the surrounding normal tissue. Images obtained such imaging can be created, in accordance with an image processing method, as a positive image obtained from negative imaging, or can be created in a reverse manner.

In one aspect, the present invention can be used for discriminating a cancer tissue. Cancer that can be discriminated in the present invention is not especially limited, and examples include kidney cancer, bladder cancer, esophageal cancer, stomach cancer, colorectal cancer, breast cancer, skin cancer, lung cancer, liver cancer, peritoneal cancer, peritoneal dissemination, head and neck cancer, brain cancer, or the like. Administration route and the like can be appropriately selected in accordance with the type of the cancer. For example, when bladder cancer is to be discriminated, the compound of the present invention is systemically administered by intravenous administration or the like, the bladder is irradiated with near-infrared light, and near-infrared fluorescence emitted from the compound specifically accumulated in a bladder cancer tissue is observed to discriminate the bladder cancer tissue. Alternatively, the compound of the present invention can be intravesically injected via the urethra, and thereafter, the inside of the bladder is washed if necessary, and thus, a compound not adsorbing onto a bladder cancer tissue can be washed/removed. Besides, in the present invention, the compound of the present invention can be systemically administered, or topically administered, so as to discriminate a bladder cancer tissue.

As fluorescence intensity measurement means used in discrimination of a cancer tissue in the present invention, fluorescence intensity measurement means suitable for measurement in a site where the cancer has been caused may be appropriately selected in accordance with the type of the cancer. For example, in case of bladder cancer, a bladder cancer tissue can be discriminated with a cystoscope for near-infrared fluorescence from the inside of the bladder, or a bladder cancer tissue can be discriminated with a near-infrared fluorescence observation device from outside the bladder.

### EXAMPLES

A representative embodiment of the present invention will now be specifically described with reference to the following examples, and it is noted that the present invention is not limited by the following examples but can be practiced with various modifications made within the scope of the present invention. It is herein noted that, unless otherwise stated, a concentration and the like are on a weight basis, and that a range of a numerical value includes endpoints thereof.

In the following examples, a chloride of the following compound I or compound II was used as a cyclodextrin-bonded indocyanine compound. These compounds exhibit fluorescence in a near-infrared region (700 nm to 2500 nm), particularly in the vicinity of 800 nm to 830 nm.

### - Preparation of Cyclodextrin-bonded Indocyanine Compound

### (1) Compound III (Chloride of Compound I)

58 kg of methanol, 2.6 kg of dimethylsulfoxide, 19 kg of water, 3-(2-carboxyethyl)-2-{(1E)-2-[(3E)-3-{(2E)-2-[3-(2-carboxyethyl)-1,1-dimethyl-1,3-dihydro-2H-benzo[e]indol-2-ylidene]ethylidene}-2-methoxycyclohexa-1-en-1-yl]ethene-1-yl}-1,1-dimethyl-1H-benzo[e]indol-3-ium chloride, and 21-O-(3-aminopropyl)cyclomaltoheptaose (pure content: 21.5 kg) were mixed at about 20°C.

Next, 4.68 kg of 4-(4,6-dimethoxy-1,3,5-triazine-2-yl)-4-methylmorpholinium chloride (DMT-MM) (pure content: 3.98 kg) was added thereto, the resultant was washed with a methanol aqueous solution (6.8 kg of methanol, and 2.1 kg of water), followed by stirring at about 20°C for about 2 hours. Thereafter, 2.34 kg of DMT-MM (pure content: 1.99 kg) and 0.726 kg of N-methylmorpholine were added thereto, followed by stirring at about 20°C for about 2 hours. Then, 2.34 kg of DMT-MM (pure content: 1.99 kg) was added thereto at about 20°C. To the resultant, 84 kg of methanol was added in a dropwise manner over about 1 hour, followed by stirring at about 20°C for about 16 hours. To the resultant, 250 kg of acetone was added in a dropwise manner over 2 hours, and the thus generated suspension was stirred at an internal temperature of about 20°C for about 19 hours. The resultant was filtered, and the thus obtained filter cake was washed with an acetone-methanol mixture (58 kg of acetone and 28 kg of methanol), and further washed with 84 kg of acetone twice. The resultant was dried under reduced pressure with an external temperature set to about 30°C, and thus, 24.6 kg of a green solid was obtained. This solid was dissolved in a 5 mM hydrochloric acid aqueous solution, and the resultant was purified by ODS column chromatography (ODS: 130 kg, mobile phase: 5 mM hydrochloric acid aqueous solution -> 30% methanol aqueous solution (v/v) -> 40% methanol aqueous solution (v/v) -> 80% methanol aqueous solution (v/v)). A target product was collected, and methanol was distilled off under reduced pressure to obtain a concentrate.

Next, the entire amount of the concentrate was adsorbed on and caused to flow through HP20SS column (HP20SS: 130 kg, mobile phase: 60% methanol aqueous solution (v/v) -> 80% methanol aqueous solution (v/v)) to collect an active moiety. The active moiety was concentrated under reduced pressure, and the resultant was subjected to clarification filtration, and then freeze dried to obtain 8.62 kg of a green solid. 4.9 kg of the solid was dissolved in a 1 mM hydrochloric acid aqueous solution, and the resultant was purified by ODS column chromatography (ODS: 150 kg, mobile phase: 1 mM hydrochloric acid aqueous solution -> 30% methanol aqueous solution (v/v) -> 40% methanol aqueous solution (v/v)). A target product was collected, and methanol was distilled off under reduced pressure to obtain a concentrate. Next, the entire amount of the concentrate was adsorbed on and caused to flow through HP20SS column (HP20SS: 67.8 kg, mobile phase: 60% methanol aqueous solution (v/v) -> 80% methanol aqueous solution (v/v)) to collect an active moiety, and the active moiety was concentrated under reduced pressure to obtain a concentrate. To the concentrate, activated carbon was added, followed by stirring at about 20°C for about 2 hours. The activated carbon was removed therefrom with a filter precoated with Radiolite, and the resultant was subjected to clarification filtration, and then freeze dried to obtain, as a green solid, 1.88 kg of 3-(3-{[3-(cyclomaltoheptaose-21-O-yl)propyl]amino}-3-oxopropyl)-2-{(1E)-2-[(3E)-3-{(2E)-2-[3-(3-{[3-(cyclomaltoheptaose-21-O-yl)propyl]amino}-3-oxopropyl)-1,1-dimethyl-1,3-dihydro-2H-benzo[e]indol-2-ylidene]ethylidene}-2-methoxycyclohexa-1-en-1-yl]ethene-1-yl}-1,1-dimethyl-1H-benzo[e]indol-3-ium chloride (compound III).

Physico-chemical properties of the obtained compound were as follows.

**[Table 1]**

| **Table 1 1H NMR Data** | | | |
|---|---|---|---|
| **Chemical Shift δ (ppm)** | **Multiplicity and Coupling Constant (Hz)** | **Number of Protons** | **Position No.** |
| 1.66 | m | 2 | 2, 54 |
| 1.81 | m | 2 | 2, 54 |
| 2.12 | m | 2 | 29 |
| 2.33 | s | 6 | 22, 48 ^{a)} |
| 2.44 | s | 6 | 21, 47 ^{a)} |
| 4.35 | s | 3 | 31 |
| 2.3-4.7 | - | 104 | 1, 3, 6, 7, 28, 30, 49, 50, 53, 55, 1', 1", 2', 2", 3', 3", 4', 4", 6', 6", 8', 8", 9', 9", 10', 10", 11', 11", 12', 12", 14', 14", 15', 15", 16', 16", 17', 17", 18', 18", 20', 20", 21', 21", 22', 22", 23', 23", 24', 24", 26', 26", 27', 27", 28', 28", 29', 29", 30', 30", 32', 32", 33', 33", 34', 34", 35', 35", 36', 36", 38', 38", 39', 39", 40', 40", 41', 41", 42', 42" |
| 5.00 | d | 2 | 5', 5", 7', 7" 13', 13", 19', 19", 25', 25", 31', 31", 37', 37" |
| 5.08 | d | 2 | |
| 5.10 | d | 2 | |
| 5.21 | d | 2 | |
| 5.29 | d | 2 | |
| 5.39 | d | 2 | |
| 5.43 | d | 2 | |
| 6.44 | d (J=14.4 Hz) | 2 | 23,33 |
| 7.72 | m | 4 | 14, 15, 39, 40 |
| 7.87 | d (J=8.8 Hz) | 2 | 10, 44 |
| 8.07 | m | 2 | 13, 41 |
| 8.39 | m | 2 | 16, 38 |
| 8.45 | d (J=14.4 Hz) | 2 | 24, 32 |
| 8.48 | d (J=8.8 Hz) | 2 | 11, 43 |

| | | | |
|---|---|---|---|
| s: singlet, d: doublet, m: multiplet a) Peak assignment is exchangeable. | | | |

### (2) Compound IV (Chloride of Compound II)

A chloride of the compound II (compound IV) was prepared based on a method described in Mol. Pharm. 17, 2672-2681 (2020) (CD-NIR-1).

### - Experiment 1

Two bladder cancer mouse models (weight: about 20 g), having mouse bladder cancer cell MB49 transplanted in the bladder mucosa, and then grown for 3 weeks or 4 weeks, were used. To the tail vein of each of the mice, 0.2 mL of a saline containing the compound III (concentration of the compound III: 0.075 mg/mL) was administered, and after 1 day, the mouse was subjected to laparotomy under anesthesia by subcutaneous administration of ketamine (75 mg/kg) and medetomidine (1 mg/kg), and near-infrared fluorescence observation was performed with a near-infrared fluorescence imaging device (pde-neo, manufactured by Hamamatsu Photonics K.K., excitation wavelength: 760 nm, detection wavelength: fluorescence of 800 nm or more, the same applies hereinafter) (Fig. 1, right: near-infrared fluorescence image).

As a comparative example, a saline containing the compound III was administered to the tail vein of a normal mouse (weight: about 20 g), and after 1 day, the mouse was subjected to laparotomy under anesthesia by subcutaneous administration of ketamine (75 mg/kg) and medetomidine (1 mg/kg), and near-infrared fluorescence observation was performed with a near-infrared fluorescence imaging device (pde-neo, manufactured by Hamamatsu Photonics K.K.) (Fig. 2).

In a cancer tissue of the bladder cancer mouse model, near-infrared fluorescence was specifically observed (Fig. 1, right: near-infrared fluorescence image). On the other hand, a normal tissue did not emit near-infrared fluorescence (Fig. 2, right: near-infrared fluorescence image).

### - Experiment 2

Two bladder cancer mouse models (weight: about 20 g), having mouse bladder cancer cell MB49 transplanted in the bladder mucosa, and then grown for 3 weeks or 4 weeks, were used. To the bladder of each of the mice, 0.2 mL of a saline containing the compound III (concentration of the compound III: 0.075 mg/mL) was administered via the urethra, and after 30 minutes, the inside of the bladder was washed via the urethra with saline five times. Thereafter, the mouse was subjected to laparotomy under anesthesia by subcutaneous administration of ketamine (75 mg/kg) and medetomidine (1 mg/kg), and near-infrared fluorescence observation was performed with a near-infrared fluorescence imaging device (pde-neo, manufactured by Hamamatsu Photonics K.K.) (Fig. 3, right: near-infrared fluorescence image).

As a comparative example, a saline containing the compound III was administered to the bladder of a normal mouse (weight: about 20 g) via the urethra, and after 30 minutes, the inside of the bladder was washed via the urethra with saline five times. Thereafter, the mouse was subjected to laparotomy under anesthesia by subcutaneous administration of ketamine (75 mg/kg) and medetomidine (1 mg/kg), and near-infrared fluorescence observation was performed with a near-infrared fluorescence imaging device (pde-neo, manufactured by Hamamatsu Photonics K.K.) (Fig. 4).

In a cancer tissue of the bladder cancer mouse model, near-infrared fluorescence was specifically observed (Fig. 3, right: near-infrared fluorescence image). On the other hand, a normal tissue did not emit near-infrared fluorescence (Fig. 4, right: near-infrared fluorescence image).

### - Experiment 3

Two bladder cancer model mice (weight: about 20 g), having mouse bladder cancer cell MB49 transplanted in the bladder mucosa, and then grown for 3 weeks or 4 weeks, were used. To the tail vein of each of the mice, 0.2 mL of a saline containing the compound III (concentration of the compound III: 0.075 mg/mL) was administered. One day after the administration, the mouse was subjected to laparotomy under anesthesia by subcutaneous administration of ketamine (75 mg/kg) and medetomidine (1 mg/kg), and under observation of near-infrared fluorescence with a near-infrared fluorescence imaging device (pde-neo, manufactured by Hamamatsu Photonics K.K.), a cancer tissue site emitting near-infrared fluorescence was taken out for near-infrared fluorescence observation (Fig. 5, right: near-infrared fluorescence image).

Since a cancer tissue emits fluorescence through near-infrared irradiation, a tumor tissue could be appropriately excised with the fluorescence emitted from the cancer tissue used as a guide according to the present invention.

### - Experiment 4

Two bladder cancer mouse models (weight: about 20 g), having mouse bladder cancer cell MB49 transplanted in the bladder mucosa, and then grown for 3 weeks or 4 weeks, were used. To the tail vein of each of the mice, 0.2 mL of a saline containing the compound IV (concentration of the compound IV: 0.075 mg/mL) was administered, and after 1 day, the mouse was subjected to laparotomy under anesthesia by subcutaneous administration of ketamine (75 mg/kg) and medetomidine (1 mg/kg), and near-infrared fluorescence observation was performed with a near-infrared fluorescence imaging device (pde-neo, manufactured by Hamamatsu Photonics K.K.) (Fig. 6, right: near-infrared fluorescence image).

As a comparative example, a saline containing the compound IV was administered to the tail vein of a normal mouse (weight: about 20 g), and after 1 day, the mouse was subjected to laparotomy under anesthesia by subcutaneous administration of ketamine (75 mg/kg) and medetomidine (1 mg/kg), and near-infrared fluorescence observation was performed with a near-infrared fluorescence imaging device (pde-neo, manufactured by Hamamatsu Photonics K.K.) (Fig. 7).

In a cancer tissue of the bladder cancer mouse model, near-infrared fluorescence was specifically observed (Fig. 6, right: near-infrared fluorescence image). On the other hand, a normal tissue did not emit near-infrared fluorescence (Fig. 7, right: near-infrared fluorescence image).

### - Experiment 5

Two bladder cancer mouse models (weight: about 20 g), having mouse bladder cancer cell MB49 transplanted in the bladder mucosa, and then grown for 3 weeks or 4 weeks, were used. To the bladder of each of the mice, 0.2 mL of a saline containing the compound IV (concentration of the compound IV: 0.075 mg/mL) was administered via the urethra, and after 10 minutes, the inside of the bladder was washed via the urethra with saline five times. Thereafter, the mouse was subjected to laparotomy under anesthesia by subcutaneous administration of ketamine (75 mg/kg) and medetomidine (1 mg/kg), and near-infrared fluorescence observation was performed with a near-infrared fluorescence imaging device (pde-neo, manufactured by Hamamatsu Photonics K.K.) (Fig. 8, right: near-infrared fluorescence image).

As a comparative example, a saline containing the compound IV was administered to the bladder of a normal mouse (weight: about 20 g) via the urethra, and after 10 minutes, the inside of the bladder was washed via the urethra with saline five times. Thereafter, the mouse was subjected to laparotomy under anesthesia by subcutaneous administration of ketamine (75 mg/kg) and medetomidine (1 mg/kg), and near-infrared fluorescence observation was performed with a near-infrared fluorescence imaging device (pde-neo, manufactured by Hamamatsu Photonics K.K.) (Fig. 9).

In a cancer tissue of the bladder cancer mouse model, near-infrared fluorescence was specifically observed (Fig. 8, right: near-infrared fluorescence image). On the other hand, a normal tissue did not emit near-infrared fluorescence (Fig. 9, right: near-infrared fluorescence image).

### - Experiment 6

Two bladder cancer mouse models (weight: about 20 g), having mouse bladder cancer cell MB49 transplanted in the bladder mucosa, and then grown for 3 weeks or 4 weeks, were used. To the tail vein of each of the mice, 0.2 mL of a saline containing the compound IV (concentration of the compound IV: 0.075 mg/mL) was administered, and 1 day after the administration, the mouse was subjected to laparotomy under anesthesia by subcutaneous administration of ketamine (75 mg/kg) and medetomidine (1 mg/kg), and under observation of near-infrared fluorescence with a near-infrared fluorescence imaging device (pde-neo, manufactured by Hamamatsu Photonics K.K.), a cancer tissue site emitting near-infrared fluorescence was taken out for near-infrared fluorescence observation (Fig. 10, right: near-infrared fluorescence image).

Since a cancer tissue emits fluorescence through near-infrared irradiation, a tumor tissue could be appropriately excised with the fluorescence emitted from the cancer tissue used as a guide according to the present invention.

### - Experiment 7

A kidney cancer mouse model was created based on a method reported by Okada et al., (Okada et al., Jpn. Arch. Int. Med., 1982, 29, 485). Specifically, rats (280 g to 320 g) were bred after continuously intraperitoneally administering ferric nitrilotriacetate to the rats for 6 months, and thus, renal epithelial cell carcinoma was induced therein.

Each of the rats was anesthetized by intraperitoneally administering pentobarbital sodium salt, a saline containing the compound III was administered to the tail vein (dose of the compound: 0.75 mg/kg of the rat), and 30 minutes, 3 hours, or 24 hours after the administration, the rat was subjected to laparotomy to perform near-infrared fluorescence imaging of the kidney with a near-infrared fluorescence imaging device (PDE, manufactured by Hamamatsu Photonics K.K.) (Fig. 11). Besides, the kidney having been subjected to the near-infrared fluorescence imaging was excised from the body, and the cross section was subjected to near-infrared fluorescence imaging (Fig. 12). Three rats each were used at each of the above-described timings after the administration, and nine rats were used in total.

In near-infrared fluorescence macro images of the surface of the kidneys having tumor and pretumor due to the continuous intraperitoneal administration of ferric nitrilotriacetate, strong fluorescence was locally found in a dotted manner, which seemed to be not the epidermis of the kidney but a cortex portion inside the epidermis (enlarged photograph in a lower portion of Fig. 11).

Besides, also in a near-infrared fluorescence image of the cross section of the kidney, a site exhibiting strong fluorescence in a dotted manner was observed in the renal cortex including the glomerulus and the renal tubule (enlarged photograph in a lower portion of Fig. 12), but such dotted fluorescence was not observed in the renal medulla (particularly, inner zone of the renal medulla not including the renal tubule).

The aspect of the fluorescence through the near-infrared irradiation was not observed in normal kidney, and is an aspect of the fluorescence peculiar to the kidney of the rat having ferric nitrilotriacetate administered. It is noted that near-infrared fluorescence was not observed with the same fluorescence measurement sensitivity in the kidneys of a normal rat and a rat having ferric nitrilotriacetate administered to which the compound III was not administered. It was revealed, based on these test results, that a site imaged with a high fluorescence intensity was present in the renal cortex of the kidney having tumor and pretumor due to ferric nitrilotriacetate administration.

Besides, a specimen for microscopic observation was created through ethanol immobilization, section creation, hematoxylin/eosin staining for performing observation in bright field and near-infrared fluorescence (Fig. 13). Specifically, when a micro image of a tissue section of the kidney having tumor due to ferric nitrilotriacetate administration was obtained with a near-infrared fluorescence microscope, strong near-infrared fluorescence was found locally in a non-neoplastic degeneration site, a pre-neoplastic degeneration site, and a neoplastic degeneration site. Thus, tumor embedded in surrounding tissues could be detected. A macro fluorescence image and a micro fluorescence image of these showed abnormal aspect of fluorescence in a pretumor site and a tumor site.

### - Experiment 8

A syngeneic tumor transplanted mouse model was created as follows referring to "Gan - Shikkan Model no Sakusei to Riyo (Cancer - Creation and Use of Disease Model)" (Takuro Nakamura, LIC, 2012).

(Breast Cancer) A tumor model was created by breeding a normal Balb/c mouse (weight: about 20 g to 30 g) for 14 days with mouse tumor cell 4T1 subcutaneously administered thereto.

(Colorectal Cancer) A tumor model was created by breeding a normal Balb/c mouse (weight: about 20 g to 30 g) for 14 days with CT26 (colorectal cancer cell) subcutaneously administered thereto.

(Skin Cancer) A tumor model was created by breeding a C57BL/6 mouse (weight: about 15 g to 20 g) for 14 days with B 16F 1 (skin cancer cell) subcutaneously administered thereto.

Subsequently, to the tumor of each of the model mice, 0.02 to 0.05 mL of a saline containing the compound III or ICG (concentration of the compound III or ICG: 1 mg/mL) was administered, and fluorescence imaging was performed transdermally with a near-infrared fluorescence camera (Fluobeam, manufactured by Fluoptics, http://www.solve-net.com/info/wp-content/uploads/downloads/2013/12/Fluobeam.pdf) (excitation wavelength: 780 nm, detection wavelength: 800 nm or more).

Fluorescence images of the respective tumor models are illustrated in Figs. 14 to 17. It was confirmed, through the fluorescence imaging, that the compound III having been administered into the tumor was retained in the tumor, and hence the appearance of the tumor could be imaged without leaking a fluorescence signal to around the tumor (Fig. 14: breast cancer, Fig. 15: colorectal cancer, and Fig. 16: skin cancer).

Besides, when fluorescence imaging was conducted during a tumor excision operation visually performed, a state where the tumor isolated from the mouse and picked up with tweezers was clearly fluorescence imaged was observed, and therefore, it was confirmed that the administered compound did not exude to surrounding tissues but the outline of the tumor was drawn (Fig. 17). Besides, a part of the tumor tissue exhibiting a fluorescence signal was left in the mouse tissues, and thus, a residual tumor tissue insufficiently excised through the visual tumor excision could be confirmed, and it was presumed that there was a possibility of further excision of the tissue. On the other hand, when ICG was administered, fluorescence signals were emitted outside the tumor tissue, and the outline of the tumor could not be drawn.

### - Experiment 9

Metastatic spontaneous renal cell carcinoma line from BALB/c mouse, RenCa cell (1 x 10⁶ cells, purchased from CLS Cell Lines Service GmbH, Eppelheim, Germany) was prepared in RPMI-1640 medium (fetal bovine serum free, FUJIFILM Wako Pure Chemical Corporation), the resultant was inoculated in a mouse (BALB/cCrSlc, SPF, female, 7 weeks old) by tail vein administration under anesthesia by subcutaneous administration of ketamine (75 mg/kg) and medetomidine (1 mg/kg), and the resultant mouse was bred for 18 days to cause lung cancer therein. The mouse was anesthetized by subcutaneous administration of ketamine (75 mg/kg) and medetomidine (1 mg/kg), and the compound III (120 nmol/kg weight) was administered through the tail vein. After 10 minutes, the lung was excised to perform near-infrared fluorescence imaging with a near-infrared fluorescence imaging device (pde-neo, manufactured by Hamamatsu Photonics K.K.). It was found that the intensity of near-infrared fluorescence of the compound III was low in a lung cancer site (framed site in the near-infrared fluorescence image) (Fig. 18).

In this manner, it was revealed that a site of a lung cancer tissue can be specified as a portion having a low intensity of the near-infrared fluorescence of the compound III.

### - Experiment 10

Metastatic spontaneous renal cell carcinoma line from BALB/c mouse, RenCa cell (1 x 10⁶ cells, purchased from CLS Cell Lines Service GmbH, Eppelheim, Germany) was intravenously inoculated into a mouse (BALB/cCrSlc, SPF, female, 7 weeks old) under anesthesia by subcutaneous administration of ketamine (75 mg/kg) and medetomidine (1 mg/kg), and the resultant mouse was bred for 18 days to cause liver cancer therein. The mouse was anesthetized by subcutaneous administration of ketamine (75 mg/kg) and medetomidine (1 mg/kg), and the compound III (120 nmol/kg weight) was administered through the tail vein. After 10 minutes, laparotomy was conducted to perform near-infrared fluorescence imaging with a near-infrared fluorescence imaging device (pde-neo, manufactured by Hamamatsu Photonics K.K.). Near-infrared fluorescence of the compound III from a liver normal tissue was strong, light emission of the compound III from a liver cancer site (framed site in the right image) was very weak, and thus, the normal site and the cancer site could be distinguished from each other depending on the light emission (Fig. 19).

In this manner, it was revealed that a site of a liver cancer tissue can be specified based on the fluorescence of the compound III.

### - Experiment 11

Human stomach cancer cell MKN45 (1 x 10⁷ cells, purchased from JCRB cell bank of National Institutes of Biomedical Innovation, Health and Nutrition) was subcutaneously transplanted into the right or left side, or the center of the back in the base of the foreleg of a nude mouse (BALB/cSlc-nu, SPF, male, 5 weeks old), and the resultant mouse was bred for 10 days to create a mouse having a stomach cancer tissue grown therein. After anesthetizing the mouse (weight: about 20 g) by subcutaneous administration of ketamine (75 mg/kg) and medetomidine (1 mg/kg), the compound III (120 nmol/kg weight) was administered through the tail vein, and near-infrared fluorescence was observed from above the skin on the back side with a near-infrared fluorescence imaging device (pde-neo, manufactured by Hamamatsu Photonics K.K.). The near-infrared fluorescence was stronger in a cancer tissue than in a normal tissue, and thus, the cancer tissue (site surrounded by a dotted line) and the normal tissue could be distinguished from each other (Fig. 20).

In this manner, it was revealed that a stomach cancer tissue and a normal tissue can be discriminated from each other in accordance with a difference in near-infrared fluorescence intensity obtained through observation of the near-infrared fluorescence of the compound III.

### - Experiment 12

Metastatic spontaneous renal cell carcinoma line from BALB/c mouse, RenCa cell (5 x 10⁶ cells, purchased from CLS Cell Lines Service GmbH, Eppelheim, Germany) was intraperitoneally inoculated into a mouse (BALB/cCrSlc, SPF, female, 7 weeks old) and the resultant mouse was bred for 14 days to cause peritoneal cancer therein. The mouse (weight: about 20 g) was anesthetized by subcutaneous administration of ketamine (75 mg/kg) and medetomidine (1 mg/kg), and the compound III (12 nmol/kg weight) was administered through the tail vein. Immediately after the administration, laparotomy was conducted to perform near-infrared fluorescence imaging with a near-infrared fluorescence imaging device (pde-neo, manufactured by Hamamatsu Photonics K.K.). Near-infrared fluorescence of the compound III from a peritoneal cancer site (framed site in the near-infrared fluorescence image) was stronger than that from a normal tissue, and thus, the peritoneal cancer site could be shown with the light emission (Fig. 21).

In this manner, it was revealed that a site of a peritoneal cancer tissue can be specified based on the fluorescence of the compound III.

### - Experiment 13

Human stomach cancer cell MKN45-Luc (5 x 10⁶ cells, purchased from JCRB cell bank of National Institutes of Biomedical Innovation, Health and Nutrition) was intraperitoneally injected into a nude mouse (BALB/cSlc-nu, SPF, male, weight: about 20 g), and the resultant mouse was bred for 15 days to create a nude mouse having peritoneal dissemination. After anesthetizing this mouse (cancer mouse) and a normal nude mouse (BALB/cSlc-nu, SPF, male, 5 weeks old) by subcutaneous administration of ketamine (75 mg/kg) and medetomidine (1 mg/kg), the compound III (40 nmol/kg weight) was administered through the tail vein, and near-infrared fluorescence of the compound III after the administration was observed with a near-infrared fluorescence imaging device (pde-neo, manufactured by Hamamatsu Photonics K.K.). The near-infrared fluorescence was not emitted from the abdominal cavity of the normal mouse (Fig. 22). In the cancer mouse, a visually recognizable cancer tissue surrounded with a dotted line in the right portion of Fig. 23 was nearly in a necrotic state, and hence did not emit the near-infrared fluorescence, but dissemination present in the entire abdominal cavity emitted the near-infrared fluorescence (Fig. 23).

In this manner, it was revealed that peritoneal dissemination originating from stomach cancer can be imaged by detecting the near-infrared fluorescence of the compound III.

### - Experiment 14

Under anesthesia by subcutaneous administration of ketamine (75 mg/kg) and medetomidine (1 mg/kg), RenCa kidney cancer cell (5 x 10⁶ cells, purchased from CLS Cell Lines Service GmbH, Eppelheim, Germany) was transplanted into the left kidney of a mouse (weight: about 20 g), and the resultant mouse was bred for 9 to 14 days to create a kidney cancer mouse. After anesthetizing the mouse by subcutaneous administration of ketamine (75 mg/kg) and medetomidine (1 mg/kg), the compound III (12 nmol/kg weight) was administered through the tail vein, and after 10 minutes, near-infrared fluorescence of the kidney was observed with a near-infrared fluorescence imaging device (pde-neo, manufactured by Hamamatsu Photonics K.K.). The fluorescence from a cancer tissue was weak, the fluorescence from a normal tissue was strong, and thus, the cancer tissue and the normal tissue could be distinguished from each other (Fig. 24).

In this manner, it was revealed that a kidney cancer tissue and a normal tissue can be distinguished from each other by detecting the intensity of the near-infrared fluorescence of the compound III.

### - Experiment 15

Human esophageal cancer cell KYSE850 was subcutaneously transplanted into the neck of a nude mouse (BALB/cSlc-nu, SPF, male, 5 weeks old), and the resultant mouse was bred for 18 days to create a mouse having esophageal cancer grown therein. The human esophageal cancer cell KYSE850 was purchased from JCRB cell bank of National Institutes of Biomedical Innovation, Health and Nutrition (Registration Number: JCRB1422, establisher: Graduate School of Pharmaceutical Sciences, Kyoto University, Department of Nanobio Drug Discovery, Yutaka Shimada).

### (Literatures)

Shimada Y. Imamura M, Wagata T, Yamaguchi N and Tobe T. Characterization of Twenty One Newly Established Esophageal Cancer Cell Lines. Cancer, 69: 277-284 (1992). Tanaka H, Shibagaki I, Shimada Y, Wagata T, Imamura M, Ishizaki K. Characterization of p53 gene mutations in esophageal squamous cell carcinoma cell lines: increased frequency and different spectrum of mutations from primary tumors. Int J Cancer. 65:372-376 (1996). Tanaka H, Shimada Y, Imamura M, Shibagaki I, Ishizaki K. Multiple types of aberrations in the p16 (INK4a) and the p15(INK4b) genes in 30 esophageal squamous-cell-carcinoma cell lines. Int J Cancer. 70:437-442 (1997).

Subsequently, after anesthetizing the mouse by subcutaneous administration of ketamine (75 mg/kg) and medetomidine (1 mg/kg), the compound III (120 nmol/kg weight) was administered through the tail vein, and near-infrared fluorescence of the compound III was observed with a near-infrared fluorescence imaging device (PDE, manufactured by Hamamatsu Photonics K.K.). The near-infrared fluorescence from a cancer tissue (circled site) was strong, and that from a normal tissue was weak (Fig. 25). It is noted that fluorescence in an elliptical frame corresponds to near-infrared fluorescence from the right and left kidneys.

In this manner, it was revealed that an esophageal cancer tissue and a normal tissue can be distinguished from each other by detecting the near-infrared fluorescence of the compound III.

### - Experiment 16

A human cancer cell transplant model mouse was created as follows:
(Head and neck/tongue cancer) To immunodeficient SCID mice (weight: 17 g to 23 g), about 10⁶ human tongue cancer cells CAL27 (purchased from American Type Culture Collection: ATCC) were subcutaneously administered, and the resultant mice were grown for 49 days to create tumor models.
(Brain cancer/neuroblastoma) To immunodeficient SCID mice (weight: 17 g to 23 g), about 10⁶ human neuroblastoma cells SH-SY5Y (purchased from ATCC) were subcutaneously administered, and the resultant mice were grown for 90 days to create tumor models.
(Brain cancer/glioblastoma) To immunodeficient SCID mice (weight : 17 g to 23 g), about 10⁶ human glioblastoma cells U-251MG (obtained from European Collection of Authenticated Cell Cultures) were subcutaneously administered, and the resultant mice were grown for 90 days to create tumor models.

Subsequently, to the tumor of each of the mouse models, 0.02 to 0.1 mL of a saline containing the compound III (concentration of the compound III: 1 mg/mL) was topically administered, and fluorescence imaging was conducted transdermally with a near-infrared fluorescence camera (Fluobeam, manufactured by Fluoptics) (excitation wavelength: 780 nm, detection wavelength: 800 nm or more).

Fluorescence images obtained from the respective tumor models immediately after the administration or 50 minutes to 1 hour after the administration are illustrated in Figs. 26 to 28. It was confirmed, through the fluorescence imaging, that the compound III having been administered into the tumor was retained inside the tumor, and hence the appearance of the tumor can be imaged without leaking a fluorescence signal to around the tumor (Fig. 26: tongue cancer, Fig. 27: neuroblastoma, and Fig. 28: glioblastoma).

### INDUSTRIAL APPLICABILITY

When a cyclodextrin-bonded indocyanine compound represented by (formula A) or a pharmaceutically acceptable salt thereof is administered to a subject, and near-infrared fluorescence generated through near-infrared irradiation is observed, a cancer tissue of cancer, such as bladder cancer, kidney cancer, stomach cancer, liver cancer, lung cancer, peritoneal dissemination, peritoneal cancer, esophageal cancer, breast cancer, colorectal cancer, skin cancer, head and neck cancer, and brain cancer, can be discriminated from a normal tissue, and thus, an accurate site of the cancer tissue can be identified during surgery. Accordingly, the cyclodextrin-bonded indocyanine compound represented by (formula A) or a pharmaceutically acceptable salt thereof is useful as a contrast agent for cancer.

## Claims

1. A composition for contrast imaging of cancer, comprising a cyclodextrin-bonded indocyanine compound represented by the following formula, or a pharmaceutically acceptable salt thereof: 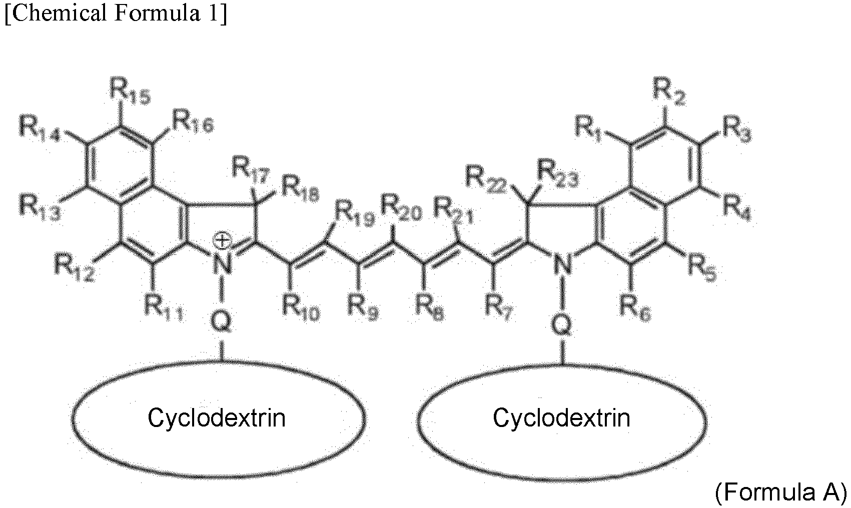
wherein two Qs are each capable of being independently selected, are optionally the same or different, and are each * 1-(CHz)a-CO-NH-(CHz)b-*3, wherein a is an integer of 2 or more and 6 or less, b is an integer of 2 or more and 6 or less, * 1 in each Q is N of the indocyanine skeleton, and *3 is a bonding portion formed by replacing any one of three OH groups of any D-glucose contained in cyclodextrin with -O-;
R1 to R23 each independently are a hydrogen atom, an alkyl group, an aryl group, a halogen atom, an alkoxyl group, an amino group, a carboxyl group, a formyl group, a sulfonyl group, a sulfonic acid group, a phosphoric acid group, an alkyloxycarbonyl group, an aryloxycarbonyl group, an alkylcarbonyl group, an arylcarbonyl group, or a heterocycle, R1 to R23 each independently are unsubstituted or substituted with a substituent, when the substituent is carboxylic acid, sulfonic acid, or phosphoric acid, a hydrogen ion is optionally dissociated from the substituent to be replaced with a metal ion;
R8 and R9 optionally together form a cyclic structure of CH₂, CH₂CH₂, CH₂CH₂CH₂, or CH₂CH₂CH₂CH₂, and one or more hydrogen atoms of the cyclic structure is optionally substituted with an aryl group, a halogen atom, an alkoxy group, an amino group, a carboxyl group, a formyl group, a sulfonyl group, a sulfonic acid group, a phosphoric acid group, an alkyloxycarbonyl group, an aryloxycarbonyl group, an alkylcarbonyl group, an arylcarbonyl group, or a heterocycle.

2. The composition according to claim 1,
wherein the cyclodextrin-bonded indocyanine compound is a compound represented by the following formula, or a pharmaceutically acceptable salt thereof: 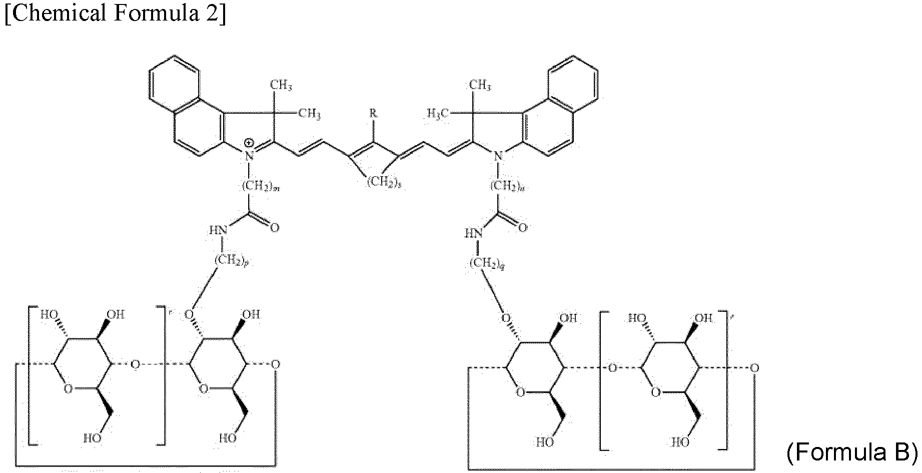
wherein m, n, p, and q are each independently an integer of 2 or more and 6 or less, r is an integer of 5 or more and 7 or less, s is an integer of 0 or more and 4 or less, and R is a hydrogen atom, an alkyl group, an aryl group, a halogen atom, an alkoxy group, an amino group, a carboxyl group, a formyl group, a sulfonyl group, a sulfonic acid group, an alkyloxycarbonyl group, an aryloxycarbonyl group, an alkylcarbonyl group, an arylcarbonyl group, or a heterocycle.

3. The composition according to claim 1 or 2, wherein the cyclodextrin-bonded indocyanine compound is a compound represented by the following formula, or a pharmaceutically acceptable salt thereof: 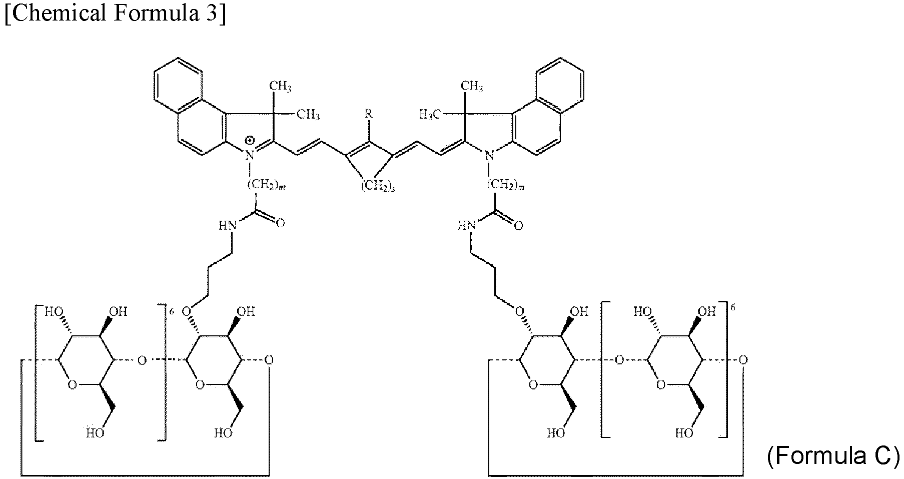 wherein m and n are each independently an integer of 2 or more and 6 or less, s is an integer of 0 or more and 4 or less, and R is a hydrogen atom, an alkyl group, an aryl group, a halogen atom, an alkoxy group, an amino group, a carboxyl group, a formyl group, a sulfonyl group, a sulfonic acid group, an alkyloxycarbonyl group, an aryloxycarbonyl group, an alkylcarbonyl group, an arylcarbonyl group, or a heterocycle.

4. The composition according to any one of claims 1 to 3, wherein the cyclodextrin-bonded indocyanine compound comprises a compound represented by the following formula, or a pharmaceutically acceptable salt thereof: 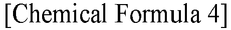 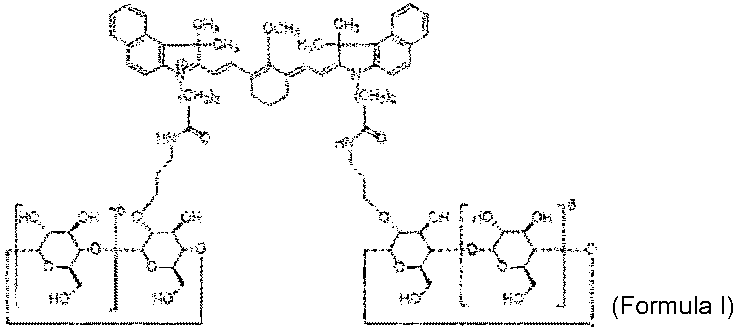 or

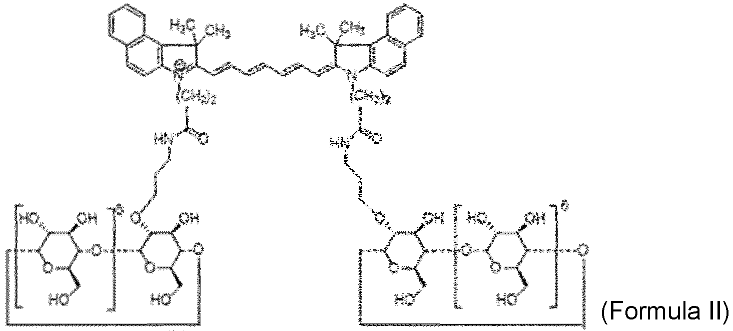

5. The composition according to any one of claims 1 to 4, in the form of a liquid.

6. The composition according to any one of claims 1 to 5, for use in imaging bladder cancer, kidney cancer, lung cancer, liver cancer, stomach cancer, peritoneal cancer, peritoneal dissemination, or esophageal cancer.

7. The composition according to any one of claims 1 to 5, for use in imaging breast cancer, colorectal cancer, skin cancer, head and neck cancer, or brain cancer.

8. The composition according to any one of claims 1 to 6, for systemic administration.

9. The composition according to any one of claims 1 to 5, and 7, for topical administration.

10. The composition according to any one of claims 1 to 5, for use in imaging bladder cancer, and for intravesical administration via the urethra.

11. The composition according to any one of claims 1 to 5, for use in imaging bladder cancer, kidney cancer, lung cancer, liver cancer, stomach cancer, peritoneal cancer, peritoneal dissemination, or esophageal cancer, and for systemic administration.

12. The composition according to any one of claims 1 to 5, for use in imaging breast cancer, colorectal cancer, skin cancer, head and neck cancer, or brain cancer, and for topical administration.

13. A cancer imaging method, comprising administering the composition according to any one of claims 1 to 5 to a subject.

14. The cyclodextrin-bonded indocyanine compound or a pharmaceutically acceptable salt thereof according to any one of claims 1 to 4, for imaging cancer.

15. Use of the cyclodextrin-bonded indocyanine compound or a pharmaceutically acceptable salt thereof according to any one of claims 1 to 4 for imaging cancer.

16. Use of the cyclodextrin-bonded indocyanine compound or a pharmaceutically acceptable salt thereof according to any one of claims 1 to 4 for producing a composition for contrast imaging of cancer.
